# EUROPÄISCHE PATENTSCHRIFT

(11) **EP 0 774 939 B1**
(45) Veröffentlichungstag und Bekanntmachung des Hinweises auf die Patenterteilung: **22.08.2001**
(21) Anmeldenummer: 94918353.7
(22) Anmeldetag: 20.05.1994
(51) Int. Cl.: A61F 5/04

(54) **VORRICHTUNG ZUR UMSCHLIESSENDEN FIXIERUNG VON EXTREMITÄTEN UND EXTREMITÄTENBEREICHEN**
DEVICE FOR SURROUNDING AND SECURING MEMBER EXTREMITIES AND REGIONS THEREOF
DISPOSITIF PERMETTANT D'ENTOURER ET DE FIXER DES EXTREMITES ET DES ZONES D'EXTREMITES DE MEMBRES

(30) Priorität: 21.05.1993 DE 9307550 U; 21.05.1993 DE 9307549 U; 21.05.1993 DE 9307548 U; 13.11.1993 DE 9317421 U; 12.03.1994 DE 9404210 U
(43) Veröffentlichungstag der Anmeldung: 28.05.1997
(73) Patentinhaber: Habermeyer, Peter, Dr., 70184 Stuttgart (DE)
(72) Erfinder: Habermeyer, Peter, Dr., 70184 Stuttgart (DE)
(74) Vertreter: Richter, Joachim, Dipl.-Ing.
(86) Internationale Anmeldenummer: EP9401648
(87) Internationale Veröffentlichungsnummer: WO9427529

(56) Entgegenhaltungen:
- EP-A- 0 355 930
- EP-A- 0 538 695
- WO-A-86/05087
- WO-A-92/04880
- WO-A-93/24081
- DE-A- 3 228 753
- US-A- 3 955 565
- US-A- 4 029 090
- US-A- 4 803 975

## Beschreibung

Die Erfindung betrifft eine Vorrichtung zur umschließenden Fixierung von Extremitäten und Extremitätenbereichen im Bereich von Unterschenkel und Oberschenkel, bestehend aus einem Schalenkörper aus zwei gegeneinander verspannbaren L-förmigen Schalenteilen mit einem etwa halbkreisförmigen Querschnittsprofil, die die zu umschließende Extremität umgeben, wobei zwischen den Schalenteilen und der Extremität mindestens ein evakuierbares Manschettenkissen vorgesehen ist, in dessen zwischen den beiden Kissenwänden gelegenem Innenraum eine Vielzahl von relativ zueinander beweglichen Füllkörpern vorgesehen ist.

Zur Behandlung von Extremitätenfrakturen werden überlicherweise Gipsverbände oder deren moderne Varianten in Form von Kunststoffverbänden angelegt. Das Anlegen derartiger Verbände erfordert nicht nur ein erhebliches Geschick und einen entsprechenden Aufwand für das Personal, sondern führt häufig auch dazu, daß durch Kanten oder Vorsprünge an der Innenseite des Gips- oder Plastikverbandes für den Patienten äußerst störende Druckstellen entstehen. Nach der Reposition der jeweiligen Fraktur ist der Patient der schmerzhaften Phase des Nachwickelns der Binden und des Wartens auf das Abbinden des Gipses ausgesetzt. Schließlich ist es bei den bekannten Gips- oder Plastikverbänden im Regelfall erforderlich, einen Wechsel dieses Gips- oder Plastikverbandes vorzunehmen, wenn es im Laufe der Fakturbehandlung zu einer Abschwellung der betreffenden Gliedmaßen kommt. Auch dies ist ein aufwendiger und für den Patienten wiederum unangenehmer Vorgang.

So ist durch die WO 92/04880 eine Vorrichtung zur umschließenden Fixierung von Extremitäten und Extremitätenbereichen bekannt, die insbesondere zur Behandlung von Extremitätenfrakturen im Bereich von Unterschenkel, Oberschenkel, Unterarm und Oberarm vorgesehen ist. Diese Vorrichtung weist zumindest zwei gegeneinander verspannbare Schalenteile auf, die die zu schließende Extremität umgeben. Zwischen den Schalenteilen und der Extremität sind zwei formbare, vakuumdicht ausgebildete und mit zumindest einem Ventil versehene evakuierbare Kissen vorgesehen, in denen sich eine Vielzahl von relativ zueinander beweglichen, insbesondere kleinkörnigen Füllkörpern befindet.

Diese bekannten Gips- und Plastikverbände setzen eine vorgegebene Winkelstellung zwischen dem Fuß und dem Unterschenkel voraus, wobei der Normalwinkelstellung der Gelenke mit einem Winkel von 90° der Vorzug gegeben wird. Nach erfolgtem Anlegen des Gipsverbandes ist eine Veränderung der Winnkelstellung nicht möglich. Wird dagegen ein Operationsgips bei Achillessehnennaht angelegt, dann wird in der Weise vorgegangen, daß nach der Operation der Unterschenkel senkrecht aufgestellt und auf maximale Spitzfußstellung angehoben wird. Auch bei einem geschlossenen Spitzfußgips nimmt der Fuß zum Unterschenkel keine Normalwinkelstellung mit einem Winkel von 90° ein, sondern die Stellung des Fußes zum Unterschenkel erfolgt in einem Winkel von größer als 90°; oftmals in einem Winkel von 120°, so daß bei einem Spitzfußgips diese Winkelstellung des Fußes zum Unterschenkel vorgegeben und festgelegt ist. Auch hier ist eine Änderung der Winkelstellung nicht möglich.

Um hier Abhilfe zu schaffen, ist es bereits vorgeschlagen worden, zwischen dem Fußteil und dem Schaftteil eines jeden Schalenteils eine Winkeleinstelleinrichtung anzuordnen, mittels der die genannten Teile in einer vorgegebenen, aber frei wählbaren Winkelstellung arrtierbar sind.

Der Nachteil der Gipsverbände sowie der vorgenannten Vorrichtungen liegt darin, daß sie entsprechend der geforderten Stabilität noch ein relativ hohes Gewicht besitzen und praktisch keine Luftzufuhr zu den umhüllten ExtremitätenBereichen Erlauben. Ein weiterer Nachteil der vorbeschriebenen Vorrichtung besteht darin, daß die Schalenteilinnenmaße konstruktiv festgelegt sind und ein Ausgleich im Hinblick unterschiedlicher Extremitätendurchmesser nur über Kissen oder sonstige Füllkörper geschaffen werden kann.

Durch die US-A-3 955 565 ist eine Vorrichtung zur umschließenden Fixierung von Extremitäten bekannt, die aus einem Schalenkörper aus zwei gegeneinander verspannbaren Schalenteilen besteht, denen es an einer Gitterstruktur der Schalenteilwandflächen fehlt, sondern die mit einer reihenweisen Lochung versehen sind. Durch diese Lochung der Schalenteile wird zwar erreicht, daß eine Belüftung der eingeschlossenen Extremität möglich ist, jedoch wird eine hohe Steifigkeit beibehalten, da die senkrecht und quer zu den einzelnen Schalenteilen verlaufenden stegartigen Abschnitte entweder relativ breit bemessen sind, um dadurch einen starren Schalenkörper mit einer hohen Eigensteifigkeit zu erhalten oder diese Stege sind so dünn, daß ein starrer Körper nicht mehr erhalten wird, wodurch keine hohe Eigensteifigkeit mehr gegeben ist. Außerdem weisen die Schalenteile keine ausreichende Elastizität auf, um ein Verspannen der beiden Schalenteile miteinander vornehmen zu können. Des weiteren ist die Lochung nur im Schaftbereich der beiden Schalenteile vorgesehen; sie erstreckt sich nicht bis in den abgewinkelten Fußteil.

Es ist daher Aufgabe der vorliegenden Erfindung, hier Abhilfe und eine gattungsgemäße Vorrichtung zur schaffen, die trotz hoher Eigensteifigkeit ein geringes Gewicht und eine gute Luftzufuhr aufweist, die ferner eine individuelle Anpassung an unterschiedliche Extremitätendurchmesser und insbesonder an die Wade des Patienten ermöglicht und universell für verschiedene Winkelstellungen des Fußes zum Unterschenkel einsetzbar ist.

Diese Aufgabe wird durch die im Anspruch 1 beschriebene Vorrichtung gelöst. Hiernach sind erfindungesgemäß die Wandflächen des Schalenkörpers gitter- und/oder rippenförmig ausgebildet. Der Vorteil einer solchen Konstruktion liegt in dem deutlich geringeren Gewicht und der besseren Luftzufuhr, wobei durch entsprechende Ausbildung der Gitter- oder Rippenstruktur die Stabilität und Eigensteifigkeit der Schalenkörper erhöht werden können.

Nach einer ersten erfindungsgemäßen Ausgestaltung bestehen die Gitterstreben und/oder Rippen aus Längs- und Querrippen, wobei die Gitterstreben oder Rippen eine Breite oder einen Durchmesser zweckmäßigerweise zwischen 1,5 und 3 cm haben.

Eine besonders gute Eigensteifigkeit läßt sich erzielen, wenn zumindest eine Längsrippe pro Schalenteil vorgesehen ist, die sich im wesentlichen über die gesamte Länge des Schalenteils erstreckt und die mindestens so breit oder mit einem größeren Querschnitt ausgestaltet ist wie die übrigen Längsstreben, Querstreben oder -rippen. Soweit die L-förmigen Schalenteile nicht einstückig, sondern mehrstückig aufgebaut sind, gilt entsprechendes auch für die einzelnen Teile. Vorzugsweise werden die Gitterstreben und/oder die Rippen jeweils in Abhängigkeit von den auf sie wirkenden Belastungen unterschiedlich breit und/oder mit einem unterschiedlichen Durchmesser versehen. Hierdurch lassen sich die Schalenteile funktionsgerecht anpassen, wobei die stärksten Längs- oder Querrippen oder - streben nur dort verwendet werden, wo die Belastung am größten ist, in anderen Bereichen jedoch schwächer dimensioniert werden. Hierdurch läßt sich die Gewichtsersparnis optimieren. Um auch den Übergang von den stabileren, sich über die gesamte Länge erstreckenden Längsrippen zu den schwächeren Querrippen bruchfester zu gestalten, sind die Anschlußstellen der Querrippen an die Längsrippe breiter als der sich anschließende, vorzugsweise in der Breite oder im Durchmesser verjüngend ausgebildete Querrippenteil.

Nach einer weiteren Ausgestaltung der Erfindung besitzt zumindest der rückseitige Schaftoberteil des Schalenkörpers einen Längsschlitz zur Breiten- oder Durchmesserverstellung, womit ein und derselbe Schalenkörper der individuellen Wadenform des Patienten angepaßt werden kann. Die den Längsschlitz umrahmenden Längsrippen sind über ein lösbares Verbindungselement auf den gewünschten Abstand einstellbar und fixierbar, womit eine konstruktiv einfache Lösung gefunden worden ist.

Nach einer weiteren Ausbildung der Erfindung weist das vordere Schalenteil ein zu dessen Schaft über ein Gelenk verschwenkbares Fußteil auf. Entsprechendes gilt für das rückseitige Schalenteil. Hiermit wird die MÖglichkeit gegeben, gewünschte Schwnkwinkel einstellen zu können. Für den Fall, daß der Fuß gegenüber dem Unterschenkel einer fixierten Winkelposition ruhig gestellt werden soll, ist weiterhin vorgesehen, daß das Gelenk und/oder das zweite Gelenk in jeder gewünschten Winkelstellung arretierbar ist.

Eine weitere Ausführungsform der Erfindung besteht darin, daß das zum Schaft verstellbare Fußteil des vorderen Schalenteiles des Schalenkörpers der Fixierungsvorrichtung über einen quer zur Schaftlängsrichtung verlaufenden, zieharmonikabalgartig ausgebildeten Abschnitt mit einer Anzahl von gefalteten, filmscharnierartig ausgebildeten und miteinander verbundenen Materialstreifen verbunden ist, wobei die das Fußteil und den Schaft miteinander verbindenden, seitlich an dem Schalenkörper ausgebildeten Schwenkgelenke mit Winkelarretierungseinrichtungen versehen sind. Die Verschwenkbarkeit und somit die Winkelveränderbarkeit des Fußteils zum Schaft wird mittels einer Vielzahl von parallel zueinander angeordneten Filmscharnieren erreicht, wobei dann die Feststellung des Fußteils zum Schaft in den Winkelstellungen von 90° und 120° über die Schwenkgelenke erfolgt. Der Gelenkbereich zwischen dem Fußteil und dem Schaft des vorderen Schalenkörpers wird hier durch einen materialabschnitt erreicht, der eine Breitenveränderbarkeit ermöglicht.

Bei einer bevorzugten Ausführungsform besteht die Winkeleinstelleinrichtung für das Fußteil zum Schaft des vorderen Schalenteils des Schalenkörpers der Fixierungsvorrichtung aus dem zwischen dem Fußteil und dem Schaft des vorderen Schalenkörpers angeordneten, dem Außenprofil des Schalenkörpers bzw. dem vorderen Schalenkörper entsprechend profilierten, plattenförmigen Winkeladapter, der mittels Rast- und Verriegelungseinrichtungen an dem Schaft und dem Fußteil des vorderen Schalenkörpers lösbar gehalten ist. Dieser plattenförmige Winkeladapter weist nach einer Ausführungsform eine Breite auf, aufgrund der das Fußteil zu dem Schaft des vorderen Schalenkörpers eine Winkelstellung von 90° einnimmt, so daß bei eingesetztem Winkeladapter das Fußteil zu dem Schaft in einem Winkel von 90° steht. Nach einer weiteren Ausführungsform weist der plattenförmige Winkeladapter eine Breite auf, aufgrund der das Fußteil zu dem Schaft des vorderen Schalenkörpers eine Winkelstellung von 120° einnimmt. Auf diese Weise ist es möglich, die Normalwinkelstellung des Fußes zum unteren Gelenk, nämlich die Winkelstellung von 90° zu erhalten, wohingegen bei der Anordnung eines breiteren Winkeladapters die bei 120° liegende Spitzfußstellung des Fußes zum Unterschenkel eingestellt und erhalten werden kann.

Für den Einsatz unterschiedlich bemessener Winkeladapter weist das Fußteil und der Schaft des vorderen Schalenkörpers im Verbindungsbereich vom Fußteil zum Schaft eine fensterartige Durchbrechung auf, die sich bis in den Seitenbereich des Fußteils und des Schaftes erstreckt, so daß der in die fensterartige Durchbrechung eingesetzte plattenförmige Winkeladapter diesen offenen Bereich zwischen dem Fußteil und dem Schaft überbrückt, wodurch die Winkelstellung des Fußteiles zum Schaft erhalten wird.

Die Befestigung des Winkeladapters erfolgt bevorzugterweise vermittels feststehender und verdrehbarer Verriegelungshaken und unter Verwendung von profilierten Randbereichen, wobei die verdrehbaren Verriegelungshaken als Exzenter ausgebildet sind, so daß durch die verdrehbaren, exzentrisch ausgebildeten Verriegelungshaken der Winkeladapter über die Gegenteile, d. h. über den Fußteil und den Schaft des vorderen Schalenkörpers gespannt wird, wodurch ein spielfreier Preßsitz erreicht wird. Auf diese Weise ist es möglich, mühelos durch Verwendung des entsprechend ausgebildeten Winkeladapters die eine oder andere erforderliche Winkelstellung zu erreichen.

Aufgrund der erfindungsgemäßen Ausgestaltung der Fixierungsvorrichtung ist es möglich, bei einem aus zwei gegeneinander verspannbaren Schalenteilen bestehenden Schalenkörper auch das vordere Schalenteil, bei dem das Fußteil zum Schaft eine vorgegebene, nicht veränderbare Winkelstellung einnehmen soll, der jeweils erforderlichen Winkelstellung und somit der durch das rückwärtige Schalenteil vorgegebenen Winkelstellung anzupassen. Der vordere Schalenteil besteht hiernach aus dem Schaft, dem Fußteil und dem zwischen beiden Teilen eingesetzten Winkeladapter, wodurch die Möglichkeit gegeben ist, von einer Spitzfußstellung - Winkelstellung 120° - die Fixierungsvorrichtung auf die Normalwinkelstellung - Winkelstellung 90° - zurückzustellen.

Es ist somit eine Anpassungsfähigkeit des vorderen Schalenteils an das rückwärtige Schalenteil gegeben. Im vorliegenden Fall werden lediglich zwei Winkeladaptertypen benötigt, nämlich einmal ein Winkeladapter, der die Einstellung des Winkels von 90° und ein Winkeladapter, der eine Einstellung auf 120° ermöglicht.

Mit allen Ausgestaltungen der Erfindung wird der Vorteil erreicht, daß auch Lageveränderungen, d. h. Winkelveränderungen des Fußteils nach einer bestimmten Behandlungszeit z. B. mühelos möglich sind. Es sind somit in einfachster Weise die verschiedensten Winkelstellungen fixierbar, was gegenüber den bekannten Gipsvergänden insofern besonders vorteilhaft ist, als die bekannten Gipsverbände nur die eine oder andere Winkelstellung einnehmen können, da nach Erhärten des Gipses keine Winkelveränderungen des Fußteiles mehr vorgenommen werden können.

Desweiteren sieht die Erfindung vor, daß zumindest der rückseitige Schaftoberteil des Schalenkörpers mindestens einen Längsschlitz zur Breiten- oder Durchmesserverstellung aufweist. Durch diese einfache konstruktive Maßnahme ist eine Abstandsverstellung und damit eine individuelle Anpassung an den Extremitätendurchmesser möglich.

Nach Weiterbildungen der Erfindung sind den Längsschlitz umrahmende Führungen über ein lösbares Verbindungselement auf den gewünschten Abstand einstellbar und fixierbar. Diese Ausführungsform hat den Vorteil, daß je nach Anordnung der Führungen und relativer Stellung des lösbaren Verbindungselementes eine stufenlose Einstellung des Schaftoberteildurchmessers erzielt werden kann. Die Führungen können beispielsweise derart angeordnet sein, daß sie in einem spitzen oder stumpfen Winkel auseinanderlaufen, so daß bei Längsbewegung des Verbindungselementes entlang der Führungen unterschiedliche Längsschlitzabstände im Schaftoberbereich einstellbar sind.

Aus herstellungstechnischen Gründen kann das Verbindungselement ein Schieber mit die Führungen teilweise im Umfang umgreifenden oder in die Führungen eingreifenden Randprofilierungen sein.

Um wahlweise die Durchmesserverstellung einsetzen oder hierauf verzichten zu können, überragt nach einer Weiterbildung der Erfindung eine der beiden Führungen die andere in Richtung der Schalenoberteillängsachse um eine Länge, die mindesten s so groß ist wie die Länge des Schiebers oder des Verbindungselementes. Bei Stellung des Verbindungselementes in Höhe des überragenden Teiles kann hierdurch das Verbindungselement (einseitig) befestigt sein, ohne in Eingriff mit der zweiten Führung zu stehen. Hierdurch ist die Handhabbarkeit des Verbindungselementes verbessert, da dies zur Abstandsveränderung nur über die zweite Führung geschoben werden muß. Vorzugsweise ist eine der beiden Führungen im wesentlichen bis zum Schalenoberteilrand ausgebildet.

Um den Verlust des lösbaren Verbindungselementes oder des Schiebers zu vermeiden, begrenzt ein Anschlag die Verschiebbarkeit in Richtung des Schaftoberteilrandes. Das Verbindungselement oder der Schieber ist in diesem Falle gegen ein unbeabsichtigtes Abziehen von der längeren Führung gesichert.

In einer konkreten Ausführungsform besitzt das Schaftoberteil zwei im wesentlichen parallel laufende rippen- oder wulstförmige Erhebungen, die in entsprechend geformte Nuten des Schiebers eingreifen, der längsverschiebbar, aber gegen ein seitliches Herausnehmen gesichert ist. Insbesondere kann die Führung eine Schwalbenschwanzführung sein.

Aus Gründen der Gewichtsersparnis, aber auch um die Luftzufuhr zu den umhüllten Extremitätenbereichen zu verbessern, sind die Wandflächen des Schalenkörpers zumindest teilweise gitter- und/oder rippenförmig ausgebildet. Hierbei ist es vorzugsweise möglich, zwei Längsrippen des Schaftoberteils dazu zu verwenden, daß sie als Schieberführungen genutzt werden.

Nach einer weiteren Erfindungsgemäßen Ausführungsform ist die Fixierungsvorrichtung in der Weise ausgebildet, daß von den beiden eine etwa L-Form aufweisenden Schalenteilen mit einem etwa halbkreisförmigen Querschnittsprofil und mit einem Schaft und einem Fußteil das rückwärtige Schalenteil ein zu dessen Schaftwinkel verstellbares Fußteil und in dem den Fußteil gegenüberliegenden Bandbereich eine festerartige Durchbrechung mit einem in diese eingesetzten und austauschbaren plattenförmigen Winkeladapter aufweist, der mit einer Eingrifföffnung versehen ist, in die zur Winkelfestlegung zwischen dem Fußteil und dem Schaft des rückwärtigen Schalenteiles ein Nocken eingreift, der zur Fixierung einer vorbestimmten Winkelstellung an dem Fußteil des rückwärtigen Schalenteiles angeformt ist.

Dadurch, daß das rückwärtige Schalenteil des Schalenkörpers der Fixierungsvorrichtung zur Aufnahme eines plattenförmigen Winkeladapters ausgebildet ist, besteht in Verbindung mit dem an dem Fußteil des rückwärtigen Schalenteiles angeordneten Nocken die Möglichkeit, das Fußteil zum Schaft in der jeweils erforderlichen Winkelstellung anzuordnen, festzustellen und zu halten, wobei die Winkelstellung des Fußteiles vorgegeben wird durch die Lage und Anordnung der Eingrifföffnung in dem plattenförmigen Winkeladapter. Ist die Eingrifföffnung im unteren Bereich des Winkeladapters vorgesehen und greift in die so lagevorgegebene Eingrifföffnung der Nocken des Fußteils in diese Eingrifföffnung ein, dann wird die Normalwinkelstellung des Fußes zum unteren Gelenk, nämlich die Winkelstellung von 90° erhalten, wohingegen bei einer Anordnung der Eingrifföffnung für die Nocken im oberen Bereich des Winkeladapters die bei 120° liegende Spitzfußstellung des Fußes zum Unterschenkel erhalten wird. Ist dagegen der plattenförmige Winkeladapter mit einer schlitzförmigen, sich in etwa über die gesamte Länge des Winkeladapters erstreckenden Eingrifföffnung versehen, dann besteht die Möglichkeit, die verschiedensten Winkelstellungen des Fußes zu dem Unterschenkel des Patienten einstellen zu können. Ist darüber hinaus der an dem Fußteil angeformte Nocken mit einer Feststellschraube versehen, dann ist die Möglichkeit gegeben, den in einer bestimmten Winkelstellung eingestellten Fußteil zum Schaft hin festzustellen.

Dadurch, daß in der Wand des rückwärtigen Schaftteils der plattenförmige Winkeladapter in einer fensterartigen Durchbrechung zweckmäßigerweise mittels Klemmsitz gehalten ist, ist ein müheloses Auswechseln von Winkeladaptern mit den verschiedensten Anordnungen und Lagen der Eingrifföffnungen möglich. Der weitere Vorteil besteht darin, daß auch Lageveränderungen, d. h. Winkelveränderungen des Fußteils nach einer bestimmten Behandlungszeit z. B. mühelos möglich ist. Es sind somit in einfachster Weise die verschiedensten Winkelstellungen fixierbar, was gegenüber den bekannten Gipsverbänden insofern besonders vorteilhaft ist, als die bekannten Gipsverbände nur die eine oder andere Winkelstellung berücksichtigen kann, da nach Erhärten des Gipses keine Winkelveränderungen des Fußteils mehr vorgenommen werden können.

Die Fixierungsvorrichtung ist in der Weise ausgebildet, daß von den beiden, eine etwa L-Form aufweisenden Schalenteilen mit einem etwa halbkreisförmigen Querschnittsprofil und mit einem Schaft und einem Fußteil, das vordere Schalenteil ein zu dessen Schaft winkelverstellbares Fußteil und in dem Bereich zwischen dem Fußteil und dem Schaft eine Winkeleinstelleinrichtung aufweist, vermittels der das Fußteil zum Schaft in einer vorgegebenen Winkelstellung arretierbar ist, wobei das rückwärtige Schalenteil ein zu dessen Schaft winkelverstellbares Fußteil oder ein zu dessen Schaft in einem vorgegebenen Winkel festgestelltes Fußteil aufweist.

Dadurch, daß das vordere Schalenteil des Schalenkörpers der Fixiervorrichtung zur Aufnahme eines plattenförmigen Winkeladapters als Winkeleinstelleinrichtung ausgebildet ist, ist die Möglichkeit gegeben, das Fußteil zum Schaft in der jeweils erforderlichen Winkelstellung anzuordnen und somit das Fußteil zum Schaft in dem jeweils erforderlichen Winkel festzustellen, wobei die Winkelstellung des Fußteils vorgegeben wird durch die Lage und Anordnung und Abmessung des Winkeladapters.

Bei einer bevorzugten Ausführungsform besteht die Winkeleinstelleinrichtung für das Fußteil zum Schaft des vorderen Schalenteils des Schalenkörpers der Fixierungsvorrichtung aus dem zwischen den Fußteil und dem Schaft des vorderen Schalenkörpers angeordneten, dem Außenprofil des Schalenkörpers bzw. dem vorderen Schalenkörper entsprechend profilierten, platenförmigen Winkeladapter, der mittels Rast- und Verriegelungseinrichtungen an dem Schaft und dem Fußteil des vorderen Schalenkörpers lösbar gehalten ist. Dieser plattenförmige Winkeladapter weist nach einer Ausführungsform eine Breite auf, aufgrund der das Fußteil zu dem Schaft des vorderen Schalenkörpers eine Winkelstellung von 90° einnimmt, so daß bei eingesetztem Winkeladapter das Fußteil zu dem Schaft in einem Winkel von 90° steht. Nach einer weiteren Ausführungsform weist der plattenförmige Winkeladapter eine Breite auf, aufgrund der das Fußteil zu dem Schaft des vorderen Schalenkörpers eine Winkelstellung von 120° einnimmt. Auf diese Weise ist es möglich, die Normalwinkelstellung des Fußes zum unteren Gelenk, nämlich die Winkelstellung von 90° zu erhalten, wohingegen bei der Anordnung eines breiteren Winkeladapters die bei 120° liegende Spitzfußstellung des Fußes zum Unterschenkel eingestellt und erhalten werden kann.

Für den Einsatz unterschiedlich bemessener Winkeladapter weist das Fußteil und der Schaft des vorderen Schalenkörpers im Verbindungsb ereich vom Fußteil zum Schaft eine festerartige Durchbrechung auf, die sich bis in den Seitenbereich des Fußteiles und des Schaftes erstreckt, so daß der in die fensterartige Durchbrechung eingesetzte plattenförmige Winkeladapter diesen offenen Bereich zwischen dem Fußteil und des Schaftes überbrückt, wodurch die Winkelstellung des Fußteiles zum Schaft erhalten wird.

Die Befestigung des Winkeladapters erfolgt bevorzugterweise vermittels feststehender und verdrehbarer Verriegelungshaken und unter Verwendung von profilierten Randbereichen, wobei die verdrehbaren Verriegelungshaken als Exzenter ausgebildet sind, so daß durch die verdrehbaren exzentrisch ausgebildeten Verriegelungshaken der Winkeladapter über die Gegenteile, d. h. über den Fußteil und den Schaft des vorderen Schalenkörpers gespannt wird, wodurch ein spielfreier Preßsitz erreicht wird. Auf diese Weise ist es möglich, mühelos durch Verwendung des entsprechend ausgebildeten Winkeladapters die eine oder andere erforderliche Winkelstellung zu erreichen.

Aufgrund dieser Ausgestaltung der Fixierungsvorrichtung ist es möglich, bei einem aus zwei gegeneinander verspannbaren Schalenteilen bestehenden Schalenkörper auch das vordere Schalenteil, bei dem das Fußteil zum Schaft eine vorgegebene nicht veränderbare Winkelstellung einnehmen soll, der jeweils erforderlichen Winkelstellung und somit der durch das rückwärtige Schalenteil vorgegebenen Winkelstellung anzupassen. Der vordere Schalenteil besteht hiernach aus dem Schaft, dem Fußteil und dem zwischen beiden Teilen eingesetzten Winkeladapter, wodurch die Möglichkeit gegeben ist, von einer Spitzfußstellung - Winkelstellung 120° - die Fixierungsvorrichtung auf die Normalwinkelstellung - Winkelstellung 90° - zurückzustellen. Es ist somit eine Anpassungsfähigkeit des vorderen Schalenteiles an das rückwärtige Schalenteil gegeben. Im vorliegenden Fall werden lediglich zwei Winkeladaptertypen benötigt, nämlich einmal ein Winkeladapter, der die Einstellung des Winkels von 90° und ein Winkeladapter, der eine Einstellung auf 120° ermöglicht.

Eine weitere Ausführungsform besteht darin, daß das zum Schaft verstellbare Fußteil des vorderen Schalenteiles des Schalenkörpers der Fixierungsvorrichtung über einen quer zur Schaftlängsrichtung verlaufenden, zieharmonikabalgartig ausgebildeten Abschnitt mit einer Anzahl von gefalteten, filmscharnierartig ausgebildeten und miteinander verbundenen Materialstreifen verbunden ist, wobei die das Fußteil und den Schaft miteinander verbindenden seitlich an dem Schalenkörper ausgebildeten Schwenkgelenke mit Winkelarretierungseinrichtungen versehen sind. Die Verschwenkbarkeit und somit die Winkelveränderbarkeit des Fußteils zum Schaft wird mittels einer Vielzahl von parallel zueinander angeordneten Filmscharnieren erreicht, wobei dann die Feststellung des Fußteiles zum Schaft in den Winkelstellungen von 90° und 120° über die Schwenkgelenke erfolgt. Der Gelenkbereich zwischen dem Fußteil und dem Schaft des vorderen Schalenkörpers wird hier durch einen Materialabschnitt erreicht, der eine Breitenveränderbarkeit ermöglicht.

Mit allen diesen Ausgestaltungen wird der Vorteil erreicht, daß auch Lageveränderungen, d. h. Winkelveränderungen des Fußteiles nach einer bestimmten Behandlungszeit z. B. mühelos möglich sind. Es sind somit in einfachster Weise die verschiedensten Winkelstellungen fixierbar, was gegenüber den bekannten Gipsverbänden insofern besonders vorteilhaft ist, als die bekannten Gipsverbände nur die eine oder andere Winkelstellung einnehmen können, da nach Erhärten des Gipses keine Winkelveränderungen des Fußteiles mehr vorgenommen werden können.

Eine gute Anpassung an die jeweilige Wade des Patienten wird mit einer Fixierungsvorrichtung erreicht, die aus zwei eine L-Form aufweisenden Schalenteilen, die zu einem Schalenkörper miteinander verspannbar sind und die ein etwa halbkreisförmiges Querschnittsprofil aufweisen, so daß der Unterschenkel des Patienten von beiden Seiten vermittels der beiden Schalenteile abschnittsweise umgriffen wird. Das rückwärtige Schalenteil des Schalenkörpers weist unter Ausbildung mindestens eines abbiegbaren Abschnittes vom oberen Schalenteilenrand ausgehend schlitzförmige Durchbrechungen auf, die sich über einen Bereich in Schalenteillängsrichtung derart erstrecken, daß in dem rückwärtigen Schalenteil federnd-elastische und gegeneinander verschiebbare Wandabschnitte ausgebildet werden, wobei diese Abschnitte vermittels einer Einrichtung fixierbar bzw. verspannbar sind, damit eine hohe Formstabilität bei hoher Paßgenauigkeit erreicht wird.

Dadurch, daß der rückwärtige Schalenteil des Schalenkörpers gegeneinander verschiebbare, federnd-elastische Wandabschnitte aufweist, ist dieser Schalenteil jeder Wadengröße und Abmessung anpaßbar, so daß eine Lagenfixierung des Unterschenkels des Patienten im Schalenkörper gewährleistet ist, und zwar unter Beibehaltung der Normalwinkelstellung zwischen Fuß und Unterschenkel. Die Einrichtung zur Fixierung der gegeneinander verschiebbaren und federnd-elastischen Wandabschnitte des rückwärtigen Schalenteiles kann als Spanngurt ausgebildet sein, wobei jedoch auch andersartig ausgebildete Fixierungseinrichtungen eingesetzt werden können, so z. B. Klemmeinrichtungen u. dgl. Wesentlich ist, daß in Anpassung an die jeweilige Wadenformgebung und Wadengröße des Unterschenkels des Patienten die Schalenteile des Schalenkörpers so fixiert sind, daß nicht nur eine Stabilisierung im rückwärtigen Bereich, sondern auch eine seitliche Stabilisierung erhalten wird. Desweiteren wirkt der abbiegbare Abschnitt in der Wand des rückwärtigen Schalenteiles bei einer Wadenmuskelbewegung als auf die Wade einwirkende Muskelpumpe, wodurch eine Thromboseprophylaxe erreicht wird. Wenn auf das rückwärtige Schalenteil das vordere Schalenteil aufgesetzt ist, dann gibt das vordere Schalenteil den 90° Winkel für das rückwärtige Schalenteil und somit für die Stellung des Fußes zum Unterschenkel vor. Die seitlichen Wandabschnitte des rückwärtigen Schalenteiles sind feststehend und wirken dabei als Stabilisatoren, was besonders dann vorteilhaft ist, wenn keine vordere Abdeckung, also kein vorderer Schalenteil vorgsehen ist. Bei fehlendem vorderen Schalenteil greifen die Verschlußorgane an den feststehenden Wandabschnitten des rückwärtigen Schalenteiles an. Das vordere Schalenteil dient als Stabilisator und gibt den 90° Winkel vor.

Weitere vorteilhafte Ausgestaltungen der Erfindung sind Gegenstand der Unteransprüche.

Ausführungsbeispiele der Erfindung werden nachstehend anhand der Zeichnungen näher erläutert. Es zeigen
- Fig. 1 und 2: jeweils verschiedene Ausführungsformen der erfindungsgemäßen Vorrichtung in perspektivischer Ansicht,
- Fig. 3: eine schematische, schaubildliche Ansicht des vorderen Schalenteiles der aus zwei L-förmigen Schalenteilen bestehenden Fixierungsvorrichtung mit eingesetztem Winkeladapter,
- Fig. 4: eine Seitenansicht der Fixierungsvorrichtung aus einem Schalenkörper mit zwei L-förmigen Schalenteilen,
- Fig. 5: die Fixierungsvorrichtung gemäß Fig. 4 teils in einer Seitenansicht, teils in einem senkrechten Längsschnitt,
- Fig. 6: in einer Seitenansicht die beiden L-förmigen Schalenteile des Schalenkörpers vor deren Verspannung,
- Fig. 7: in einer schaubildlichen Ansicht einen Winkeladapter für eine Winkelstellung von 90° zwischen dem Fußteil und dem Schaft des vorderen Schalenkörpers,
- Fig. 8: in einer schaubildlichen Ansicht einen Winkeladapter für eine Winkelstellung von 120° zwischen dem Fußteil und dem Schaft des vorderen Schalnkörpers,
- Fig. 9: einen Schnitt gemäß Linie IX-IX in Fig. 7,
- Fig. 10: in einer vergrößerten Wiedergabe den Verbindungsabschnitt A zwischen dem Winkeladapter und der Wandfläche des Schaftes des vorderen Schalenkörpers,
- Fig. 11 und 12: jeweils verschiedene Ausführungsformen der Vorrichtung in perspektivischer Ansicht,
- Fig. 13: eine vergrößerte Teilansicht der Schaftoberteilausbildung mit einer durch einen Schieber gegebenen Verstellmöglichkeit,
- Fig. 14: einen Schnitt entlang der Linie XIV-XIV nach Fig. 13,
- Fig. 15: eine weitere Ausführungsform eines Schiebers,
- Fig. 16: eine Draufsicht auf die Ausbildung nach Fig. 15,
- Fig. 17: eine Ansicht des rückseitigen Schalenteiles der aus zwei L-förmigen Schalenteilen bestehenden Fixierungsvorrichtung,
- Fig. 18: eine Seitenansicht der Fixierungsvorrichtung aus einem Schalenkörper mit zwei L-förmigen Schalenteilen,
- Fig. 19: die Fixierungsvorrichtung teils in einer Seitenansicht, teils in einem senkrechten Längsschnitt,
- Fig. 20: in einer Seitenansicht die beiden L-förmigen Schalenteile des Schalenkörpers vor deren Verspannung,
- Fig. 21: in einer Seitenansicht die aus einem plattenförmigen Winkeladapter und einem in diesen eingreifenden Nocken bestehende Winkeleinstelleinrichtung,
- Fig. 22: in einer Vorderansicht den plattenförmigen Winkeladapter,
- Fig. 23: eine Ansicht von oben auf den Winkeladapter gemäß Fig. 22,
- Fig. 24: eine schematische Ansicht eines Winkeladapters zur Einstellung eines der Normalwinkelstellung von 90° entsprechenden Winkels,
- Fig. 25: eine schematische Ansicht eines Winkeladapters zur Einstellung eines der Spitzfußstellung von 120° entsprechenden Winkels,
- Fig. 26: eine schematische Ansicht eines Winkeladapters mit einer Winkeleinstellmöglichkeit über einen größeren Bereich,
- Fig. 27: eine schaubildliche Ansicht des vorderen Schalenteiles der aus zwei L-förmigen Schalenteilen bestehenden Fixierungsvorrichtung mit eingesetztem Winkeladapter,
- Fig. 28: eine Seitenansicht der Fixierungsvorrichtung aus einem Schalenkörper mit zwei L-förmigen Schalenteilen,
- Fig. 29: die Fixierungsvorrichtung gemäß Fig. 28 teils in einer Seitenansicht, teils in einem senkrechten Längsschnitt,
- Fig. 30: in einer Seitenansicht die beiden L-förmigen Schalenteile des Schalenkörpers vor deren Verspannung,
- Fig. 31: in einer schaubildlichen Ansicht einen Winkeladapter für eine Winkelstellung von 90° zwischen dem Fußteil und dem Schaft des vorderen Schalenkörpers,
- Fig. 32: in einer schaubildlichen Ansicht einen Winkeladapter für eine Winkelstellung von 120° zwischen dem Fußteil und dem Schaft des vorderen Schalenkörpers,
- Fig. 33: einen Schnitt gemäß Linie XXXIII-XXXIII in Fig. 31,
- Fig. 34: in einer vergrößerten Wiedergabe den Verbindungsabschnitt A zwischen dem Winkeladapter und der Wandfläche des Schaftes des vorderen Schalenkörpers,
- Fig. 35: eine Seitenansicht der Fixierungsvorrichtung aus einem Schalenkörper mit zwei L-förmigen Schalenteilen,
- Fig. 36: die Fixierungsvorrichtung teils in einer Seitenansicht, teils in einem senkrechten Längsschnitt,
- Fig. 37: in einer Seitenansicht die beiden L-förmigen Schalenteile des Schalenkörpers vor deren Verspannung,
- Fig. 38: eine Seitenansicht der Schalenkörper,
- Fig. 39: einen waagerechten Schnitt gemäß Linie XXXIX-XXXIX in Fig. 36,
- Fig. 40: eine Seitenansicht des rückwärtigen Schalenteiles und
- Fig. 41: eine Rückansicht des rückwärtigen Schalenteiles.

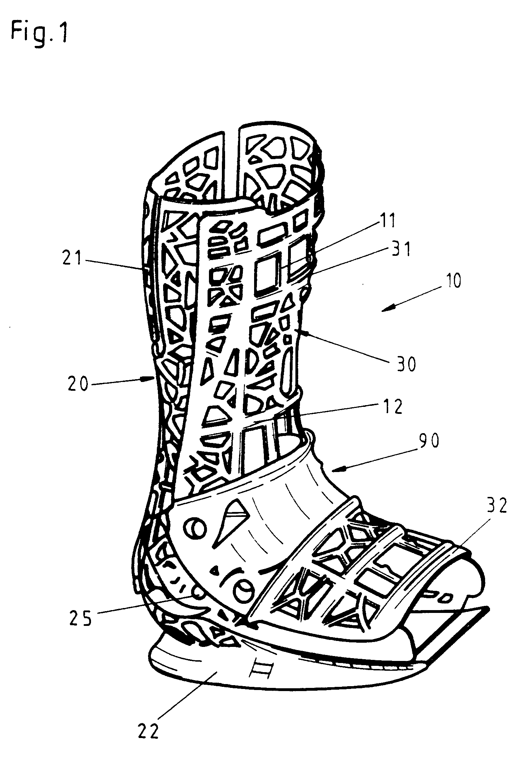

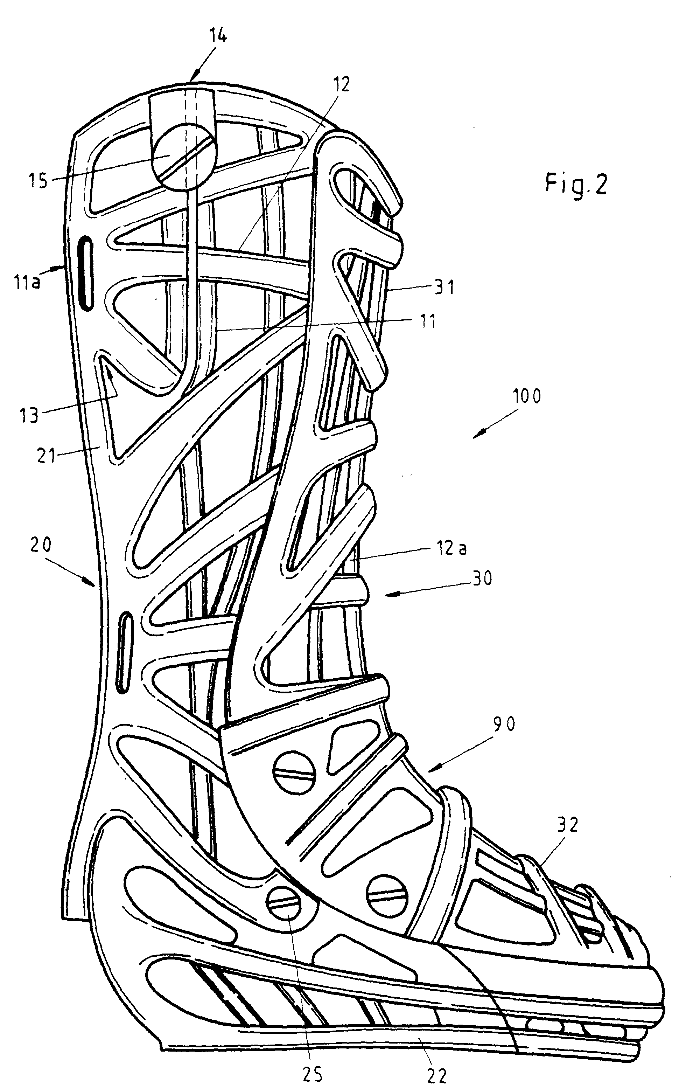

Eine Vorrichtung zur umschließenden Fixierung von Extremitäten ist in Fig. 1 und 2 als Schalenkörper 10 für den Fuß-/Unterschenkelbereich eines Patienten dargestellt. Der Schalenkörper 10 besteht aus zwei gegeneinander verspannbaren Schalenteilen, nämlich einem rückwärtigen Schalenteil 20 und einem vorderen Schalenteil 30, wobei beide Schalenteile 20, 30 in etwa eine L-Form aufweisen. Jedes Schalenteil 20 bzw. 30 besteht aus einem Schaft 21 bzw. 31 und einem Fußteil 22 bzw. 32, wobei die Fußteile 22, 32 der beiden Schalenteile mit ihren Schäften 21, 31 über seitliche Schwenkgelenke 25, 35 miteinander verbunden sind, so daß die Fußteile 22, 32 zu den Schäften 21, 31 um Schwenkachsen 25a, 35a verschwenkbar sind. Sowohl die beiden Schalenteile 20, 30 als auch ihre Schwenkgelenke 25, 35 bestehen zweckmäßigerweise aus Kunststoff.

Die Fußteile 22, 32 und die Schäfte 21, 31 der beiden Schalenkörper sind schalenförmig einseitig offen ausgebildet, so daß in dem rückwärtigen Schalenteil 20 eine Aufnahmewanne für den Fuß und den Unterschenkel geschaffen ist. In gleicher Weise ist auch der vordere Schalenkörper 30 ausgebildet, so daß dieser mit seinem Schaft 31 und seinem Fußteil 32 auf den Schaft 21 und das Fußteil 22 des rückwärtigen Schalenteiles 20 aufsetzbar ist. An der Unterseite des Fußteiles 22 des rückwärtigen Schalenteiles 20 ist eine Laufsohle 23 vorgesehen, deren äußere Gestalt an die Abrollbewegung des Fußes beim Gehen angepaßt ist. Hierzu ist die Laufsohle 23 als Gehwiege ausgebildet oder mit einem Gehstollen 24 versehen.

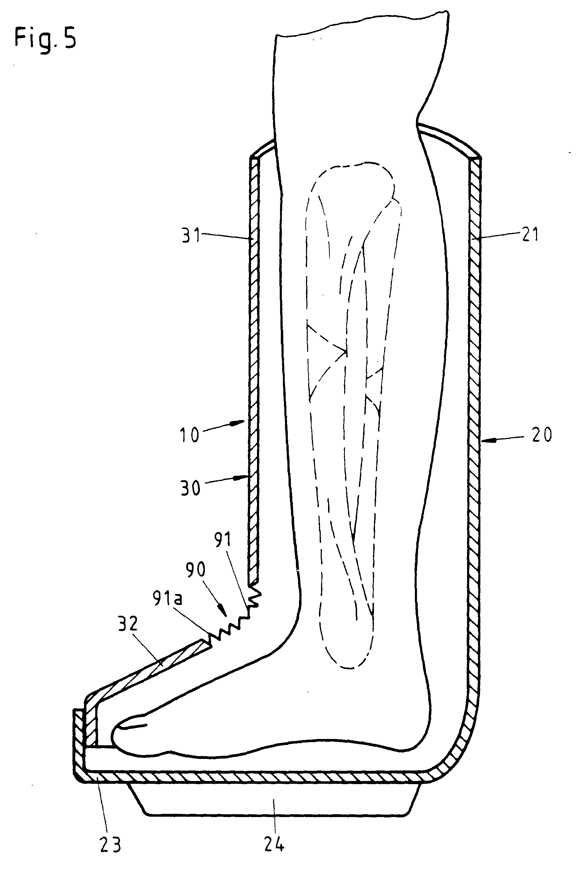

Die Fußteile 22, 23 und die Schäfte 21, 31 der beiden Schalenteile 20, 30 des Schalenkörpers 10 sind in Richtung zum Fuß und zum Unterschenkel schalenförmig ausgebildet, wobei die Ausbildung derart ist, daß das vordere Schalenteil 30 in das rückwärtige Schalenteil 20 derart einsetzbar ist, daß die seitlichen Wandabschnitte des rückwärtigen Schalenteils 20 die seitlichen Wandabschnitte des Schalenteiles 30 abschnittsweise übergreifen (Fig. 5).

Sowohl das Schalenteil 20 als auch das Schalenteil 30 weisen einen etwa halbkreisförmigen Querschnitt auf, so daß bei aufeinandergesetzten Schalenteilen ein abschnittsweises Übergreifen der Seitenwandabschnitte der beiden Schalenteile 20, 30 erfolgt. Um den Unterschenkel und auch den Fuß seitlich umgreifen zu können, weisen die Schalenteile 20, 30 eine gewisse Elastizität auf, wobei zweckmäßigerweise die Elastizität der Seitenwandungen der beiden Schalenteile 20, 30 von ihren mittigen Bereichen der Schaftteile zu ihren vorderen freien Rändern eines jeden Schalenteiles zunimmt.

Der Unterschied zwischen der in Fig. 1 dargestellten Ausführungsform zu der entsprechend Fig. 2 liegt darin, daß in Fig. 1 die Schalenteile 20 und 30 eine (engmaschigere) Gitterstruktur aufweisen als dies durch die Rippen 11, 12 bewirkt wird. Hierbei sind die Längsrippen mit 11 und die Querrippen mit 12 bezeichnet, wobei die Schaftteile 21 und 31 des rückwärtigen Teiles 20 und des vorderen Teiles 30 jeweils eine durchgehende Längsrippe 11a und 12a aufweisen, die stabiler ausgeführt sind als die Querrippen 12 und die übrigen Längsrippen. An den Anschlußstellen 13 zwischen den durchgehenden Längsrippen 11a und den Querrippen ist der Übergangsbereich breiter bzw. im Durchmesser größer ausgeführt. Hinsichtlich der Struktur, die durch Längsrippen 11, Querrippen 12 einschließlich schräg verlaufender Rippen erreichbar ist, sind keine festen Konturen vorausgesetzt, solange die erforderliche Eigensteifigkeit und Festigkeit bzw. Biegefestigkeit der Schalenteile erzielt wird. Der wesentliche Gedanke der Erfindung beruht jedenfalls darauf, daß durch gitter- oder rippenförmige Ausbildung erheblich Material eingespart werden kann und darüber hinaus die Luftzufuhr verbessert wird.

Weiterhin sind im oberen Schaftteil 21 zwei nebeneinanderliegende Längsrippen vorgesehen, die einen Spalt 14 bilden, der über ein Verbindungsmittel 15 überbrückt wird. Der Spalt 14 ist in der Breite variabel, so daß der Schaftteil den Patientenwaden angepaßt werden kann. Das rückwärtige Schalenteil 20 kann auch für sich allein zur Fixierung einer Extremität verwendet werden, die vermittels Bänder oder dgl. in dem Schalenteil gehalten und fixiert wird.

Zwischen den beiden gegeneinander verspannbaren Schalenteilen 20, 30 und der Extremität kann mindestens ein in der Zeichnung nicht dargestelltes, verformbares, vakuumdicht ausgebildetes und mit zumindest einem Ventil versehenes Kissen vorgesehen sein, das aus einer Blase besteht, in der eine Vielzahl von relativ zueinander beweglichen Füllkörpern vorgesehen ist. Das eng und passend an dem zu behandelnden Gliedabschnitt anlegbare Kissen wird bei einer innerhalb kurzer Zeit durchführbaren Evakuierung in der während des Anlegevorganges geschaffenen Form hart und formstabil, so daß in Verbindung mit den harten Schalenteilen eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entsteht und an der Innenseite auch keine Kanten oder Vorsprünge gebildet werden können. Anstelle eines derartigen Manschettenkissens kann auch ein Polster aus einem geeigneten Material eingesetzt werden; auch ein auf den Fuß und den Unterschenkel aufgezogener Strumpf kann verwendet werden.

Die an den Längsrandbereichen von Fußteil und Schaft zweckmäßigerweise vorgesehenen, in der Zeichnung nicht dargestellten Verschlußorgane, die insbesondere aus streifenförmigen Klettverschlußteilen bestehen, sorgen sehr einfach und schnell für den vor dem Evakuieren notwendigen leichten Anpreßdruck des Kissens an den geschlosenen Schalenkörper. Auf diese Weise läßt sich eine Hülse absolut korrekter Paßform erzeugen und zunächst über den Klettverschluß festlegen. nach erfolgter Evakuierung liegt dann eine Hülse vor, die in sich verfestigt und in ihrer Form stabilisiert ist und die Extremität lagegesichert hält.

Eine Bewegung im Fußgelenk des Patienten kann verhindert werden, indem der Neigungswinkel des Schaftteiles bezüglich des Fußteiles um die Schwenkachse 25 bzw. 35 fest eingestellt wird. Eine Wahl dieses fest eingestellten Winkels kann in Abhängigkeit von der zu behandelnden Verletzung innerhalb des gesamten einstellbaren Schwenkwinkelbereiches erfolgen. Das Feststellen auf einen bestimmten Winkel erfolgt mittels entsprechend ausgebildeter Verstelleinrichtungen, wobei bei dem rückwärtigen Schaftteil 20 eine Winkelvorgabe zur Einstellung eines Winkels von 90° oder von 120° zwischen dem Fußteil 22 und dem Schaft 21 vermittels eines in der Zeichnung nicht dargestellten Winkeladapters erfolgen kann.

Um eine Anpassung des vorderen Schalenteils 30 an die Winkelstellung des Fußteiles 22 zum Schaft 21 des rückwärtigen Schalenteils 20 vornehmen zu können, ist das vordere Schalenteil 30 mit einer Winkeleinstelleinrichtung 90 versehen, vermittels der das Fußteil 32 zum Schaft 31 in einer vorgegebenen Winkelstellung arretierbar ist, wobei diese Winkelstellung mit derjenigen Winkelstellung des Fußteiles 22 zum Schaft 21 des rückwärtigen Schalenteils 22 übereinstimmt.

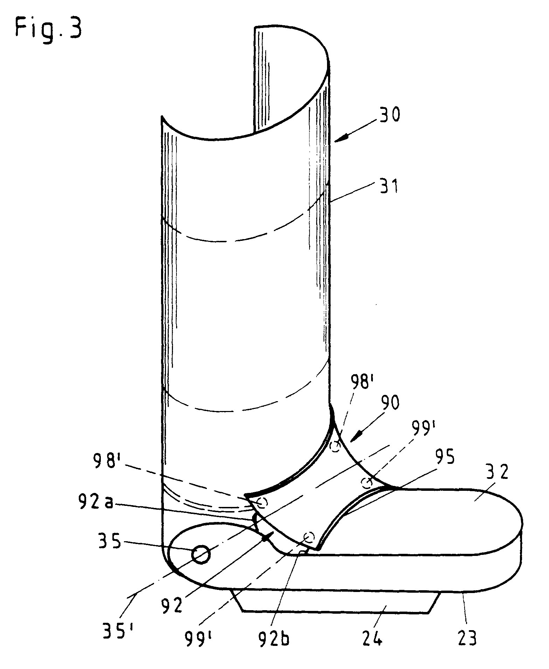

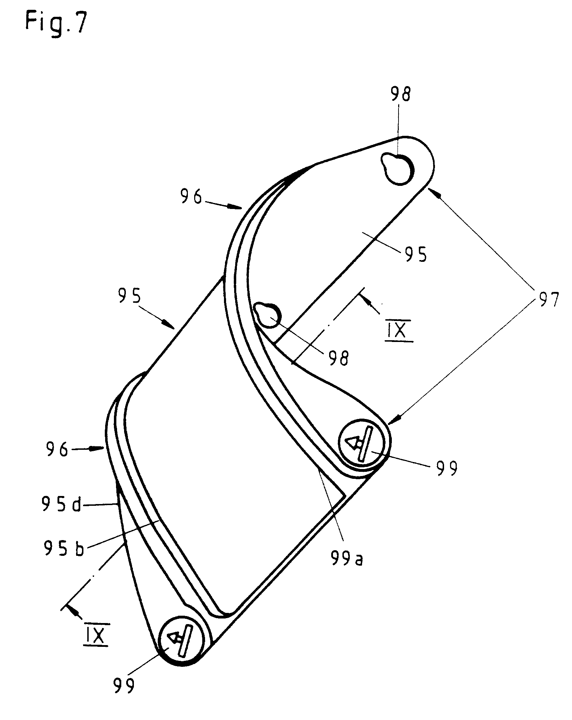

Diese Winkeleinstelleinrichtung 90 besteht aus einem zwischen dem Fußteil 32 und dem Schaft 31 des vorderen Schalenkörpers 30 angeordneten, dem Außenprofil des Schalenkörpers 10 bzw. des vorderen Schalenteils 30 entsprechend geformten, plattenförmigen Winkeladapter 95, der mittels Rast- und Verriegelungseinrichtungen 97 an dem Schaft 31 und dem Fußteil 32 des vorderen Schalenteils 30 lösbar gehalten ist (Fig. 3 und 7). Dieser Winkeladapter 95 übergreift laschenförmig vorderseitig den Verbindungsbereich 39 zwischen dem Fußteil 32 und dem Schaft 31 des vorderen Schalenkörpers 30.

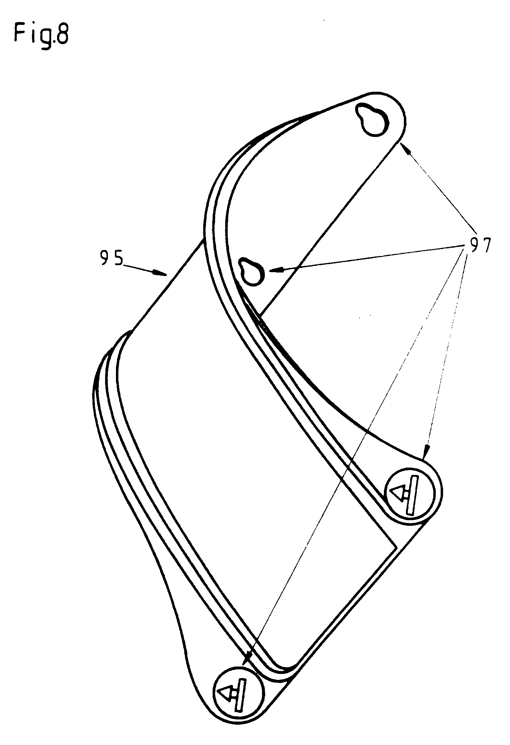

Dieser plattenförmige Winkeladapter 35 weist eine Breite auf, aufgrund der das Fußteil 32 zu dem Schaft 31 des Schalenteils 30 eine Winkelstellung von 90° einnimmt (Fig. 7). Bei dieser Winkelstellung handelt es sich um die Normalstellung zwischen dem Fuß und Unterschenkel. Eine weitere Ausführungsform des Winkeladapters 95 sieht eine Breite vor, aufgrund der das Fußteil 32 zu dem Schaft 31 des Schalenteils 30 eine Winkelstellung von 120° einnimmt, so daß, wenn diese Ausführungsform des Winkeladapters zwischen dem Fußteil 32 und dem Schaft 31 des Schalenteils 30 eingesetzt wird, das Fußteil zu dem Schaft 31 die Spitzfußwinkelstellung erhalten wird (Fig. 8).

Das Fußteil 32 und der Schaft 31 des vorderen Schalenteils 30 weist im V erbindungsbereich 39 dieser beiden Teile eine fensterartige Durchbrechung 92 auf, die sich bis etwa in den Seitenbereich des Fußteiles 32 und des Schaftes 31 erstreckt, so daß der an dem Schalenteil 30 angebrachte Winkeladapter 95 diese fensterartige Durchbrechung 92 in Form einer Abdecklasche übergreift, wobei die Abmessungen des Winkeladapters 95 derart bemessen sind, daß dieser in die fensterartige Durchbrechung 321 einsetzbar bzw. mit seinem umlaufenden Rand auf den die fensterartige Durchbrechung 92 begrenzenden Rand aufsetzbar und hier mittels Klemm- bzw. Preßsitz gehalten ist.

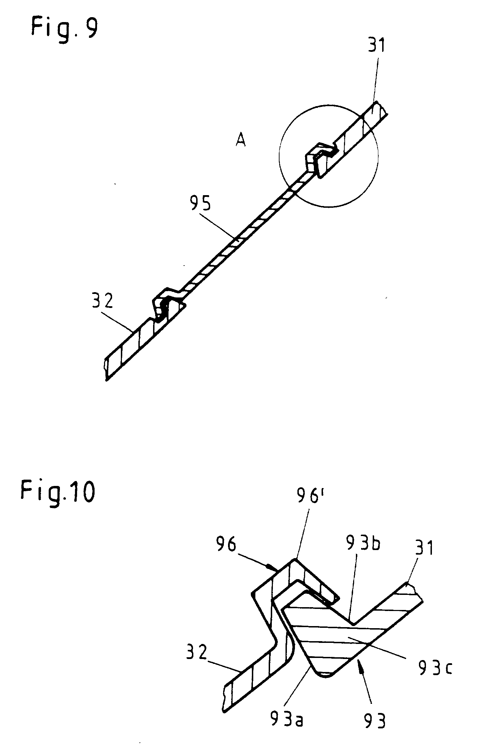

Zur Halterung des Winkeladapters 95 an den Wandflächen des Fußteiles 32 und des Schaftes 31 des Schalenteils 30 weisen die gegenüberliegenden, quer zur Schaftlängsrichtung verlaufenden Ränder 92a, 92b der fensterartigen Durchbrechung 92 außenseitig wulstartig oder andersartig profilierte, leistenförmige Verstärkungen 93 auf, während der Winkeladapter 95 an seinem oberen Rand 95a und an seinem unteren Rand 95b mit den Verstärkungen 93 an den Rändern 92a, 92b entsprechend ausgebildeten Gegenprofilen als Randprofile 96 versehen sind, so daß der Winkeladapter 95 mittels Klemmsitz an den Verstärkungen 93 an dem Fußteil 32 oder dem Schaft 31 bzw. an diesem des vorderen Schalenteils 30 gehalten ist (Fig. 9).

Die Randverstärkungen 93 an dem Fußteil 32 und dem Schaft 31 des vorderen Schalenteils 30 weisen ein Querschnittsprofil mit einer quadratischen oder rechteckförmigen, konisch nach oben verlaufende Seitenflächen 93a, 93b aufweisenden Fläche 93c auf (Fig. 10). Die Randprofile 96 an dem plattenförmigen Winkeladapter 95 sind als entsprechendes, die Randverstärkung 93 an dem Fußteil 32 und dem Schaft 31 übergreifendes Gegenprofil 96' ausgebildet.

Die Befestigung und Halterung des Winkeladapters 95 an dem Schaft 31 und dem Fußteil 32 des vorderen Schalenteils 30 erfolgt entsprechend Fig. 7 vermittels zweier an dem einen Ende 95c des Winkeladapters, und zwar innenwandseitig feststehend angeordneten Verriegelungshaken 98, die in Durchbrechungen 98' in den Wandflächen des Schaftes 31 und des Fußteils 32 des Schalenteils 30 eingreifen. Mit diesen seinem Ende 95c wird der Winkeladapter 95 in die vorgesehenen Durchbrechungen 98' in den Wandflächen des Schaftes 31 und des Fußteiles 32 des Schalenteils 30 eingehakt. An seinem anderen Ende 95d trägt der Winkeladapter 95 zwei drehbare, als Exzenter ausgebildete Verriegelungshaken 99, die ebenfalls in entsprechende Durchbrechungen 99' in den Wandflächen des Schaftes 31 und des Fußteiles 32 des Schalenteils 30 eingreifen, wobei die Verriegelung durch Verdrehen der in diese Durchbrechungen 99' eingeführten Verriegelungshaken 99 erfolgt, so daß durch diese drehbaren und als Exzenter ausgebildeten Verriegelungshaken 99 der Winkeladapter 95 über die Gegenteile, hier Fußteil 32 und Schaft 31, gespannt wird, so daß dadurch ein spielfreier Preßsitz, insbesondere an den schrägen Flächen der wulstartigen Randverstärkungen 93 erreicht wird (Fig. 10). Die Anzahl der feststehenden Verriegelungshaken 98 und der verdrehbaren Verriegelungshaken 99 kann beliebig gewählt sein. Wesentlich ist, daß jeweils ein feststehender Verriegelungshaken 98 und ein verdrehbarer Verriegelungshaken 99 miteinander korrespondieren und in die Durchbrechungen 98' im Schaft 31 und in die Durchbrechungen 99' im Fußteil 32 des Schalenteils 30 eingreifen.

Anstelle von Verriegelungshaken 98, 99 können jedoch auch andersartig ausgestaltete Befestigungsmittel eingesetzt werden.

Der plattenförmige Winkeladapter 95 besteht bevorzugterweise aus einem federnd-elastischen Material, zweckmäßigerweise aus einem Kunststoff.

Auch die beiden Schalenteile 20, 30 bestehen aus einem Kunststoff.

Die Wandflächen eines jeden Schalenteils 20 bzw. 30 sind gitterförmig unter Ausbildung von Durchbrechungen zwischen den sich kreuzenden Gitterstäben, die auch streifen- oder bandförmig ausgebildet sein können, ausgebildet. Diese Ausgestaltung erbringt den Vorteil, daß ein zwischen der Schalenkörperwand und der Extremität angeordnetes Manschettenkissen insofern lagegesichert und rutschfest angeordnet ist, als die leicht verformbaren Kissenwände in die Durchbrechungen des Schalenkörperwandgitters so gedrückt werden, daß Kissenabschnitte wulstartig aus den Durchbrechungen heraustreten bzw. in diesen zu liegen kommen und somit von den die Durchbrechungen begrenzenden Gitterstäben in ihrer Lage gehalten werden.

Eine weitere Möglichkeit der Winkelverstellbarkeit des Fußteiles 32 zum Schaft 31 des vorderen Schalenteils 30 besteht gemäß Fig. 5 darin, daß das zum Schaft 31 verstellbare Fußteil 32 des vorderen Schalenteiles 30 über einen quer zur Schaftlängsrichtung verlaufenden ziehharmonikabalgartig ausgebildeten Abschnitt 91 mit einer Anzahl von gefalteten und filmscharnierartig miteinander verbundenen Materialstreifen 91a verbunden ist, wobei die das Fußteil 32 und den Schaft 31 miteinander verbindenden, seitlich an dem Schalenkörper 10 ausgebildeten Schwenkgelenke 35 mit Winkelarretierungseinrichtungen versehen sind. Bei dieser Ausführungsform ist der sonst mittels des plattenförmigen Winkeladapters 95 verschlossenen fensterartigen Durchbrechung 92 zwischen dem Fußteil 32 und dem Schaft 31 entsprechende Bereich über einen Abschnitt 91 ausgefüllt, der aus dem gleichen Material besteht, aus dem auch der Schalenteil 30 gefertigt ist. Dieser Abschnitt 91 ist balgartig entsprechend einer Ziehharmonika ausgebildet, wobei die einzelnen Materialstreifen 91a filmscharnierartig verbunden sind, so daß in Abhängigkeit von der jeweiligen Winkelstellung des Fußteiles 32 zu dem Schaft 31 des Schalenteils 30 sich dieser dehnbare Abschnitt 91 anpaßt. Die im Bereich der Schwenkgelenke 35 vorgesehenen Winkelarretierungseinrichtungen sind in an sich bekannter Weise ausgebildet; sie bestehen aus Feststelleinrichtungen, z. B. Feststellschrauben oder dgl., so daß die vorgegebene Winkelstellung beibehalten und nur nach Lösen der Winkelarretierungseinrichtung verändert werden kann.

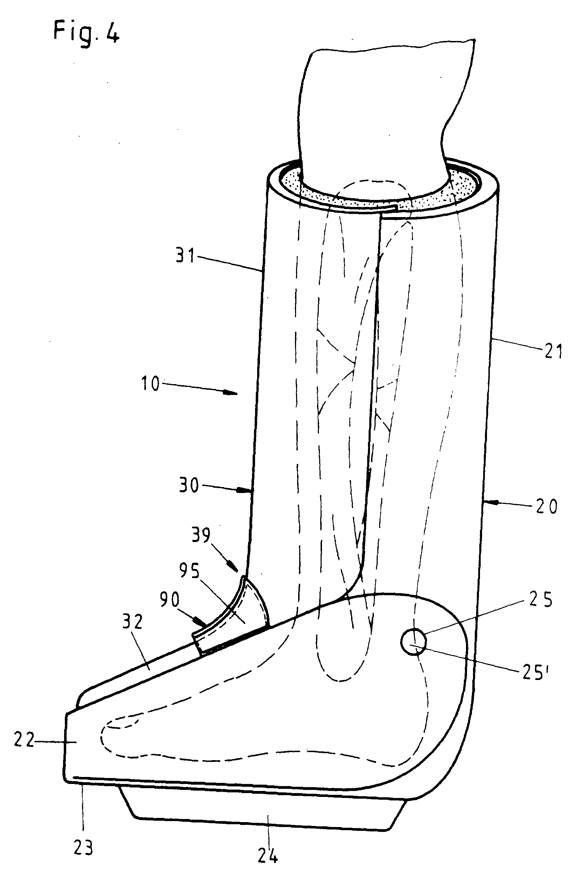

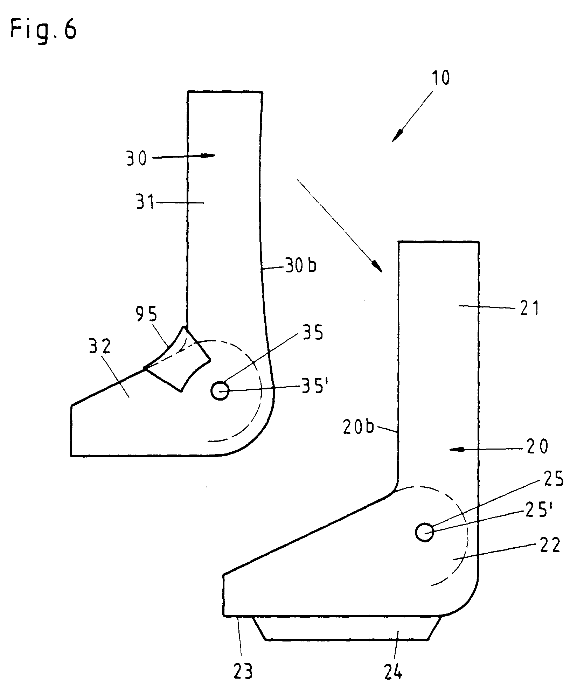

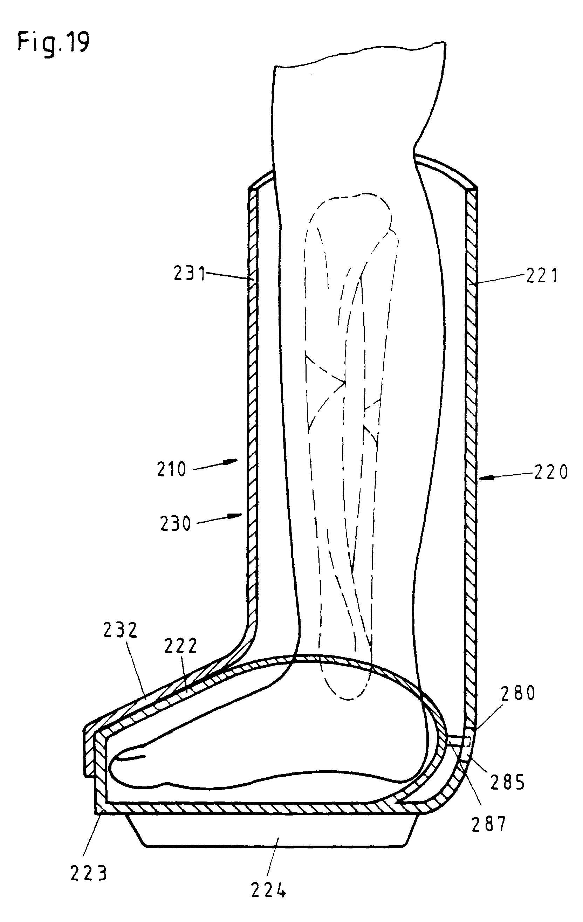

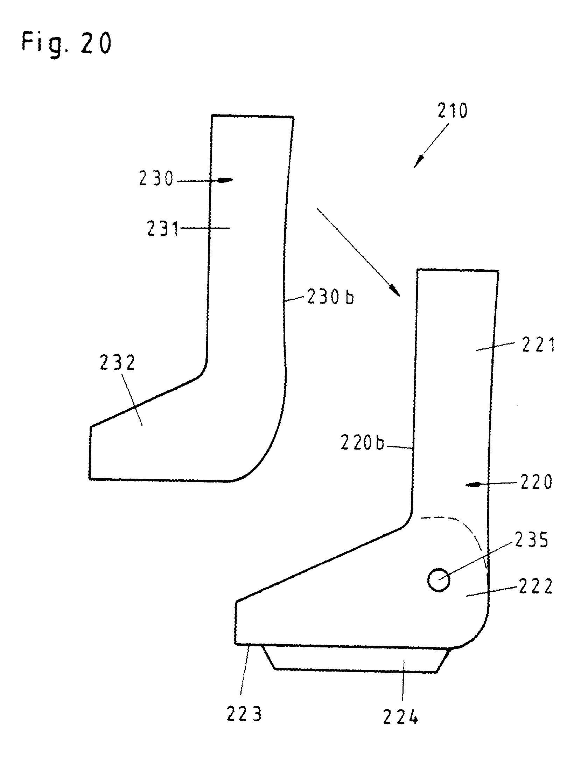

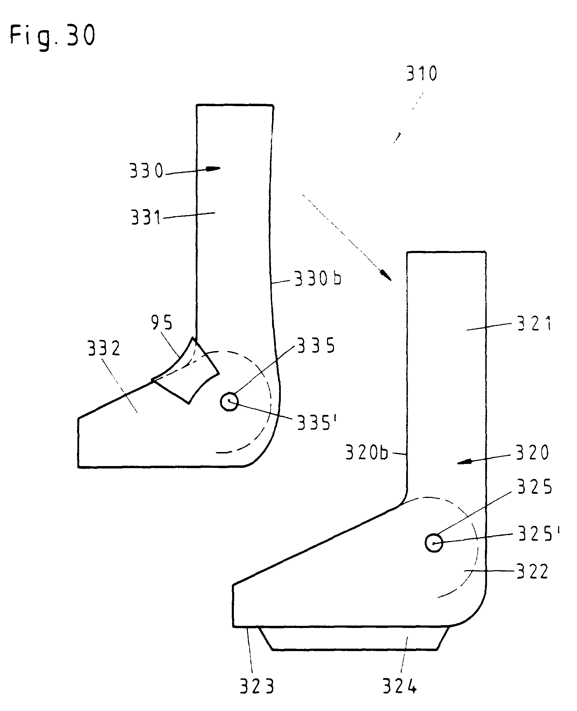

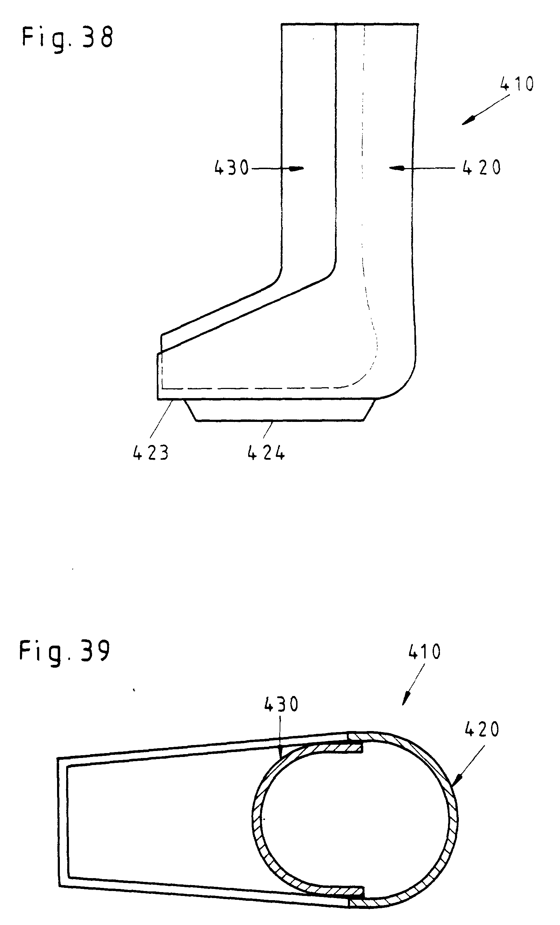

In den Fig. 3 bis 41 sind der besseren Übersicht halber die Schalenteile als Vollprofilteile abgebildet. Diese schematische Darstellung darf jedoch nicht darüber hinwegtäuschen, daß alle Schalenteile die rippen- oder gitterförmige Struktur nach Fig. 1 und 2 besitzen.

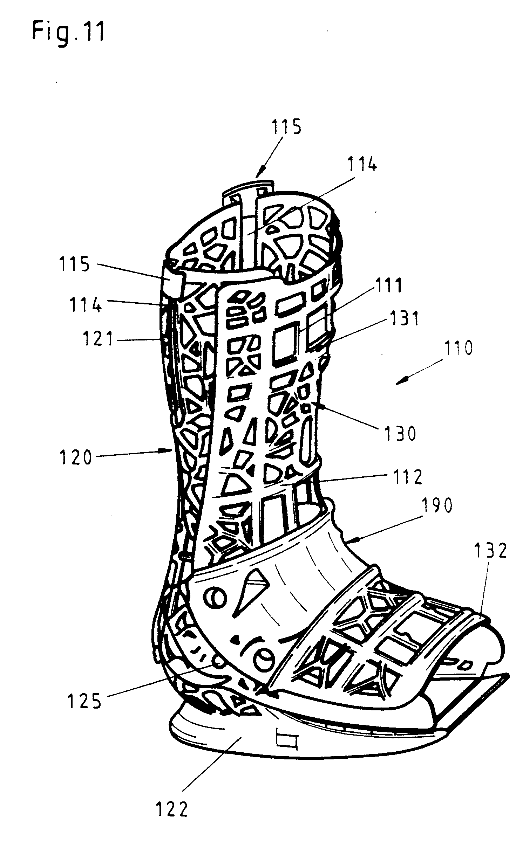

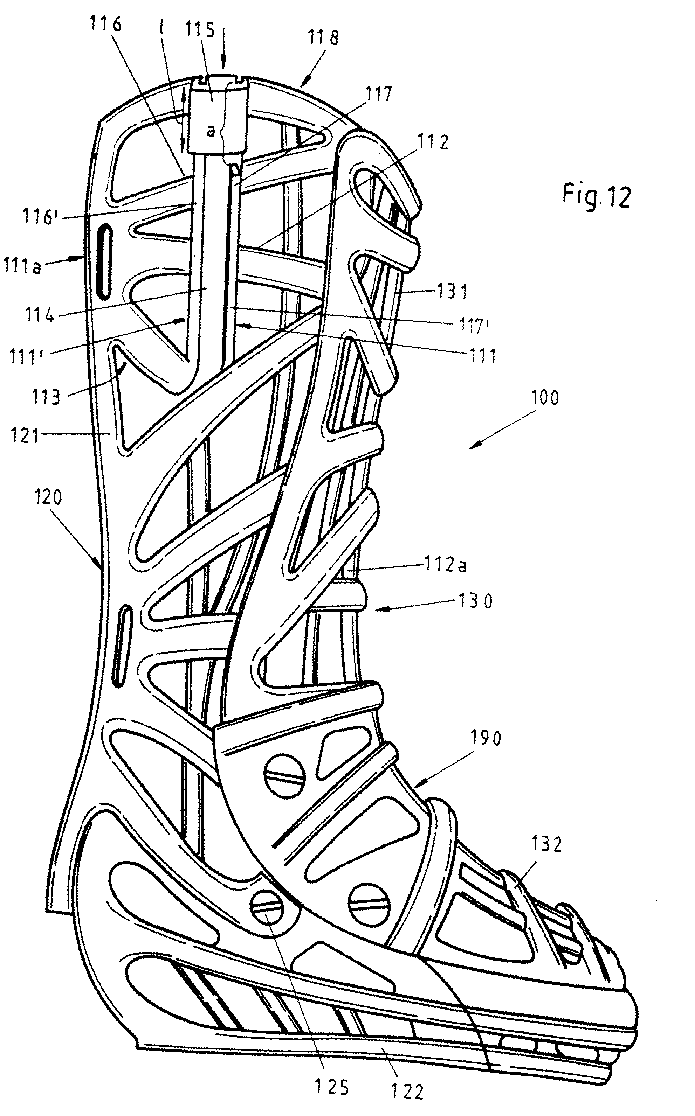

Die in Fig. 11 und 12 dargestellte Vorrichtung zur umschließenden Fixierung von Extremitäten ist in ihrer Grundkonzeption entsprechend der vorangehend beschriebenen und in Fig. 1 und 2 dargestellten Ausführungsform ausgebildet. Übereinstimmende Teile sind mit den gleichen Bezugszeichen mit einer vorangestellten "1" bezeichnet.

Im oberen Schaftteil 121 sind zwei nebeneinanderliegende Längsrippenpaare vorgesehen, die jeweils einen Spalt 114 bilden, der über ein Verbindungsmittel 115 überbrückt wird. Mindestens ein Spalt 114 ist in der Breite variabel, so daß der Schaftteil der Patientenwade angepaßt werden kann.

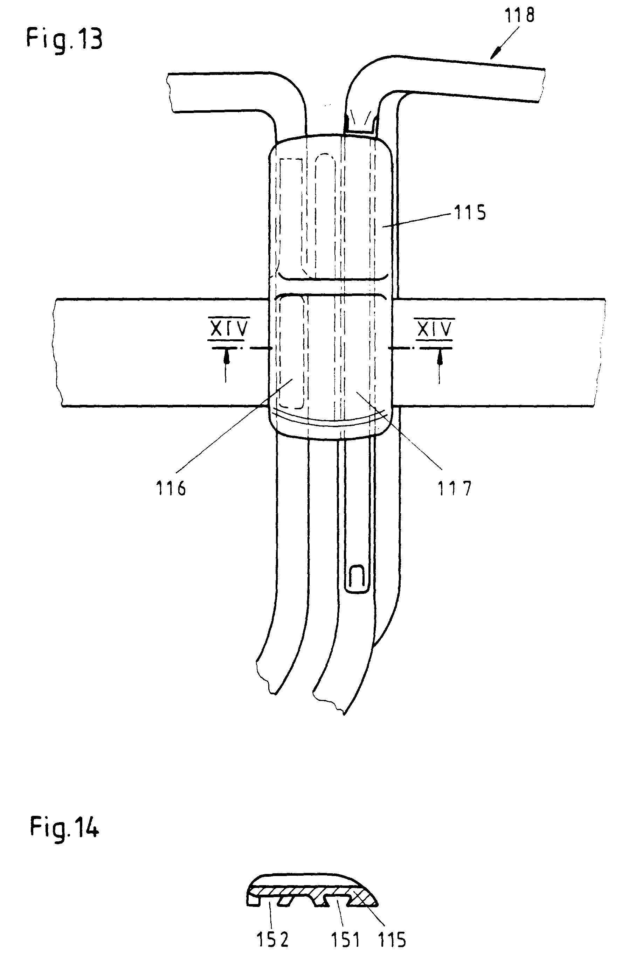

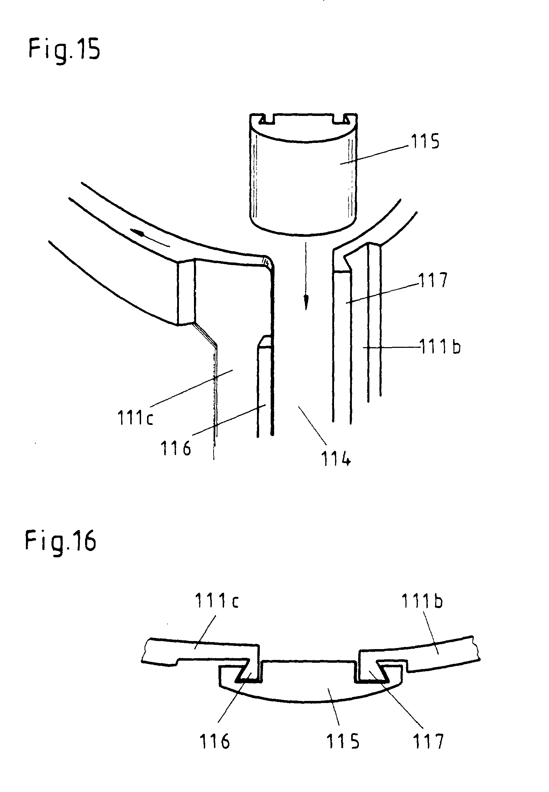

In der aus Fig. 12 und 13 näher ersichtlichen Ausführungsform dienen zwei wulstförmige, rippenförmige Erhebungen 116 und 117 auf beabstandeten Längsrippen 111 als Führungen für den Schieber 115. Die wulstförmige Erhebung 116 ist hierbei bis zum oberen Rand 118 des Schaftoberteils ausgebildet, während die Erhebung 117 in einem Abstand a darunter endet, der größer ist als die Länge 11 des Schiebers 115. Hierdurch ist es möglich, daß der Schieber 115 in der in Fig. 12 dargestellten Position zwar an dem Schalenteil 120 befestigt ist, aber noch vom Schalenteil 130 losgelöst. Wie Fig. 13 zu entnehmen ist, sind auf zwei beabstandeten Längsrippen 111, 111' Wulste als Führungsschienen 116' und 117' aufgesetzt, worüber der im Schnitt in Fig. 13 gezeigte Schieber 115 greift, und zwar dergestalt, daß die dortigen Nuten 151 und 152 ein Schwalbenschwanzprofil zeigen, das dem der wulstförmigen Erhebungen 116 und 117 der Form nach angepaßt ist. Im Unterschied zu der in Fig. 12 dargestellten Ausführungsform ist hier die Führungsschiene 117' nach unten verlängert ausgeführt, wohingegen die Schiene 16 nur eine Länge hat, die einer Teillänge des Schiebers entspricht. Die Abbildung nach Fig. 15 entspricht einer perspektivischen Abbildung der Ausführungsform nach Fig. 12. Fig. 16 zeigt den über die wulstförmigen Erhebungen 116 und 117 greifenden Verschluß zur Fixierung der gegenüberliegenden Längsrippen 111b und 111c.

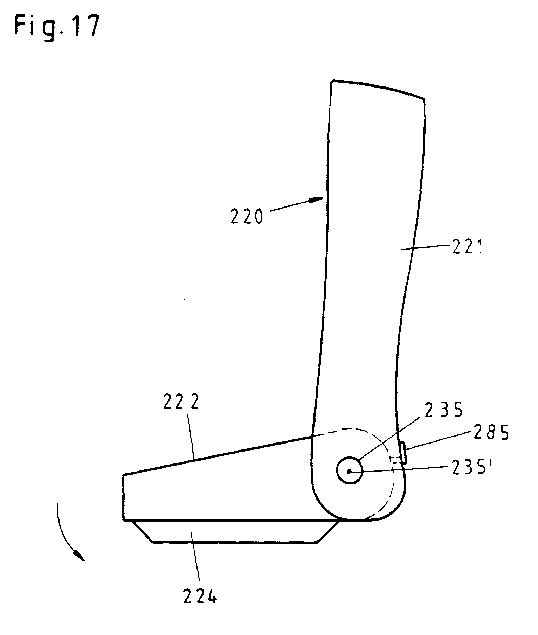

Die Vorrichtung zur umschließenden Fixierung von Extremitäten besteht gemäß Fig. 17 aus einem rückwärtigen Schalenteil 220, das eine L-Form aufweist und aus einem Schaft 221 und einem Fußteil 222 besteht, wobei das Fußteil 222 über seitliche Schwenkgelenke 235 um eine Schwenkachse 235a verschwenkbar ist, so daß der Winkel des Fußteils 222 zu dem Schaft 221 einstellbar ist. Dieses Schalenteil 220 besteht zweckmäßigerweise aus Kunststoff.

Das Fußteil 222 und der Schaft 221 des rückwärtigen Schalenteiles 220 sind schalenförmig nach oben und nach vorn offen ausgebildet, so daß eine Aufnahmewanne für den Fuß und den Unterschenkel geschaffen ist. An der Unterseite des Fußteils 222 ist eine Laufsohle 223 vorgesehen, deren äußere Gestalt an die Abrollbewegung des Fußes beim Gehen angepaßt ist. Hierzu ist die Laufsohle 223 als Gehwiege ausgebildet oder mit einem Gehstollen 224 versehen.

Vor dem Einlegen der Extremität in das rückwärtige Schalenteil 220 wird zwischen dieser und dem Schalenteil ein in der Zeichnung nicht dargestelltes verformbares, vakuumdicht ausgebildetes und mit zumindest einem Ventil versehenes Kissen eingelegt, das aus einer Blase besteht, in der eine Vielzahl von relativ zueinander beweglichen Füllkörpern vorgesehen ist. Das eng und passend an dem zu behandelnden Gliedabschnitt anlegbare Kissen wird bei einer innerhalb kurzer Zeit durchführbaren Evakuierung in der während des Anlegevorganges geschaffenen Form hart und formstabil, so daß in Verbindung mit dem harten Schalenteil 220 eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entsteht und an der Innenseite auch keine Kanten oder Vorsprünge gebildet werden können. Eine derart mit einem Manschettenkissen umhüllte und lagefixierte Extremität wird nach dem Einsetzen in das Schalenteil 220 an diesem vermittels in der Zeichnung nicht dargestellter Gurtbänder oder dgl. gehalten und lagefixiert. Anstelle eines Manschettenkissens kann auch ein Polster, ein auf die Extremität aufgezogener Strumpf oder dgl. in dem Zwischenraum vorgesehen sein.

Sollte jedoch eine die Extremität umschließende Fixierung gewünscht oder erforderlich werden, kann das rückwärtige Schalenteil 220 mit einem vorderen Schalenteil 230 zu einem Schalenkörper 210 zusammengesetzt werden, wobei auch das vordere Schalenteil 230 eine L-Form mit einem Schaft 231 und einem Fußteil 232 aufweist. Das Fußteil 232 kann zum Schaft 32 des vorderen Schalenteiles 230 feststehend und in einer vorgegebenen Winkelstellung z. B. von 90° angeordnet sein. Ein derart vorderes Schalenteil 230 wird immer dann zur Anwendung gelangen, wenn die Extremität im rückwärtigen Schalenteil 220 bei einer Winkelstellung von 90° des Fußes zum Unterschenkel des Patienten angeordnet ist. Es besteht auch die Möglichkeit, ein vorderes Schalenteil 230 einzusetzen, bei dem das Fußteil 232 zum Schaft 231 in verschiedenen Winkelstellungen angeordnet werden kann, wobei dann das Fußteil 232 in der vorgegebenen Winkelstellung arretierbar ist.

Auch das vorderseitige Schalenteil 230 ist in Richtung zum Fuß und zum Unterschenkel schalenförmig ausgebildet, wobei dann beide Schalenteile 220, 230 so ausgebildet sind, daß das vordere Schalenteil 230 in das rückwärtige Schalenteil 220 derart einetzbar ist, daß die seitlichen Wandabschnitte des Schalenteiles 220 die seitlichen Wandabschnitte des Schalenteiles 230 abschnittsweise übergreifen.

Sowohl das Schalenteil 220 als auch das Schalenteil 230 weisen einen etwa halbkreisförmigen Querschnitt auf, so daß bei ineinandergesetzten Schalenteilen ein abschnittsweises Übergriefen der Seitenwandabschnitte der beiden Schalenteile 220, 230 erfolgt. Um den Unterschenkel und auch den Fuß seitlich umgreifen zu können, weisen die Schalenteile 220, 230 eine gewisse Elastizität auf, wobei zweckmäßigerweise die Elastizität der Seitenwandungen der beiden Schalenteile 220,230 von ihren mittigen Bereichen der Schalenteile zu ihren freien Rändern eines jeden Schaftteiles zunimmt.

Die an den Längsrandbereichen von Fußteil und Schaft des rückwärtigen Schalenteiles 220 und/oder des vorderen Schaftteiles 230 zweckmäßigerweise vorgesehenen Verschlußorgane, die insbesondere aus streifenförmigen Klettverschlußteilen bestehen, sorgen sehr einfach und schnell für den vor dem Evakuieren notwendigen leichten Anpreßdruck des Kissens an den geschlossenen Schalenkörper. Auf diese Weise läßt sich eine Hülse absolut korrekter Paßform erzeugen und zunächst über den Klettverschluß festlegen. Nach erfolgter Evakuierung liegt dann eine Hülse vor, die in sich verfestigt in ihrer Form stabilisiert ist und die Extremität lagegesichert hält.

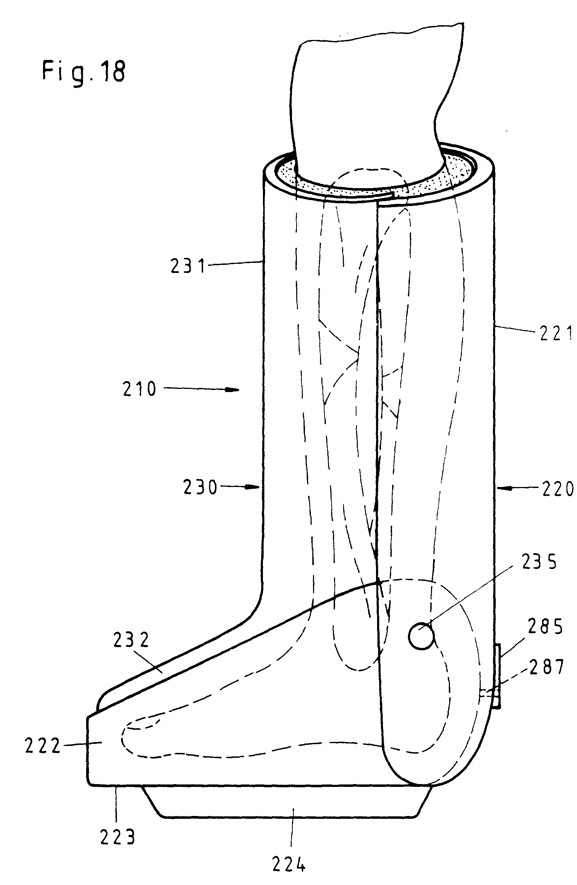

Um eine wahlweise Winkelverstellung des Fußes zum Unterschenkel bzw. des Fußteiles 222 zum Schaft 221 des rückwärtigen Schalenteiles 220 vornehmen zu können, ist in der rückwärtigen Wandfläche des Schaftes 221 des Schalenteiles 220, und zwar in dem den Fußteil 222 gegenüberliegenden Wandbereich eine fensterartige Durchbrechung 280 ausgebildet, in die ein plattenförmiger Winkeladapter 285 einsetzbar ist, der mit einer Eingrifföffnung 286 versehen ist, in die zur Winkelfestlegung zwischen dem Fußteil 222 und dem Schaft 221 des rückwärtigen Schalenteiles 220 ein Nocken 287 eingreift, der an dem Fußteil 222 des rückwärtigen Schalenteiles 220 angeformt ist (Fig. 18, 19 und 21).

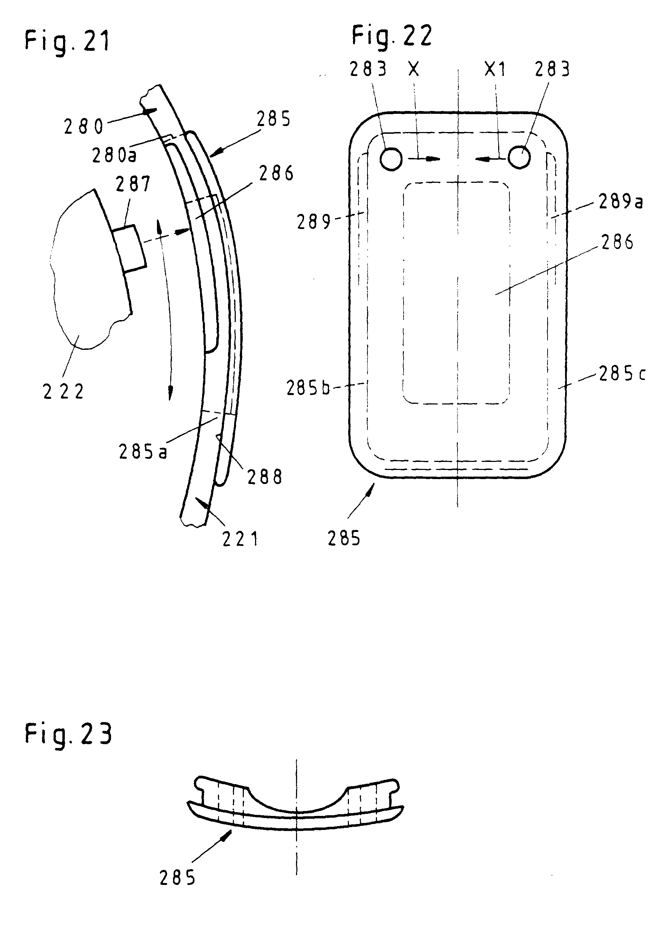

Die in diese fensterartige Durchbrechung 280 in der Rückwand des rückwärtigen Schalenteiles 220 einsetzbaren Winkeladapter 285 weisen Eingriffsöffnungen 286 für die Nokken 287 in unterschiedlichen Lagenanordnungen zur Winkeladapterfläche auf, um den Fußteil 222 zum Schaft 221 des rückwärtigen Schalenteiles 220 in vorgegebenen und bestimmten Winkelstellungen fixieren zu können (Fig. 22 und 23).

Die fensterartige Durchbrechung 280 in der Wand des rückwärtigen Schalenteiles 220 ist etwa rechteckförmig ausgebildet. Der Winkeladapter 285 weist eine der Form und den Abmessungen dieser fensterartigen Durchbrechung 280 entsprechende Ausgestaltung auf, um beispielsweise mittels Klemmsitz in der fensterartigen Durchbrechung 280 gehalten zu werden. Die Länge der Eingrifföffnung 286 in dem Winkeladapter 285 entspricht nach einer Ausführungsform einem einstellbaren Winkelbereich des Fußteils 222 zu dem Schaft 221 des Schalenteiles 220.

Die Querschnittsform des Nockens 287 kann quadratisch, rechteckförmig oder kreisförmig sein oder eine andere geometrische Form aufweisen. Die Eingrifföffnung 286 weist eine der Querschnittsform des Nockens 287 entsprechende Form auf.

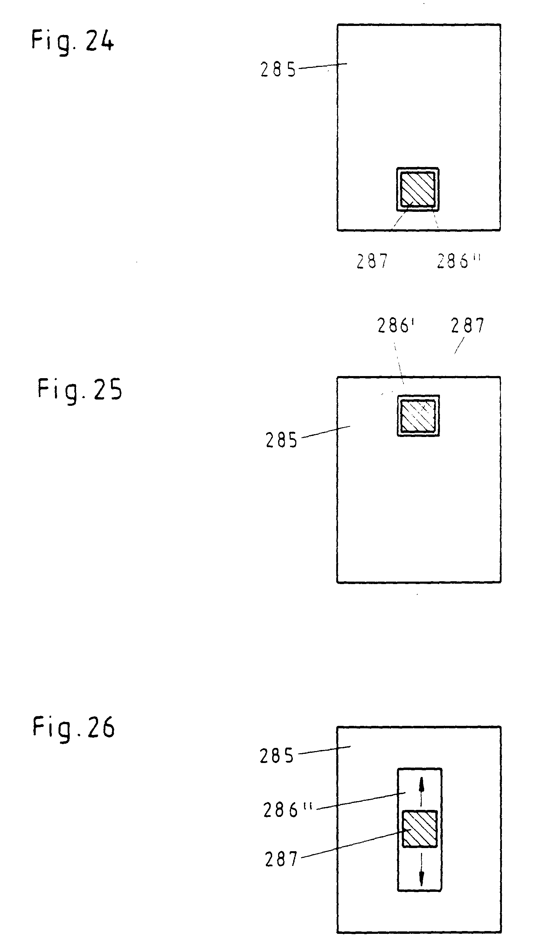

Um Winkeladapter 285 für die verschiedensten Winkelbereiche des Fußteils 222 zu dem Schaft 221 des Schalenteiles 220 zur Verfügung zu haben, ist nach einer Ausführungsform die Eingrifföffnung 286'' für den Nocken 287 im unteren Bereich des Winkeladapters 285 ausgebildet (Fig. 24). Bei dieser Ausführungsform wird eine Winkelstellung des Fußteils 222 zu dem Schaft 221 des Schalenteiles 220 in einem Winkel von 90° erhalten, was der Normalwinkelstellung zwischen dem Fuß und dem Unterschenkel entspricht.

Soll eine Spitzfußstellung von 120° als Winkel zwischen dem Fußteil 222 und dem Schaft 221 erhalten werden, dann findet ein Winkeladapter 285 verwendbar, bei dem die Eingrifföffnung 286' für die Nocken 287 im oberen Bereich des Winkeladapters 285 ausgebildet ist (Fig. 25).

Um in einem größeren Winkelbereich variabel sein zu können, kann der Winkeladapter 285 auch eine sich über die Adapterlänge erstreckende zweckmäßigerweise schlitzförmige Eingrifföffnung 286''' für den Nocken 287 aufweisen, wobei es jedoch bei dieser Ausführungsform dann vorteilhaft ist, wenn der Nocken 287 beispielsweise als Feststellschraube ausgebildet ist (Fig. 26).

Werden z. B. drei Winkeladapter 285 mit verschiedenen Anordnungen der Eingrifföffnungen 286', 286'' und 286''' zur Verfügung gestellt, so ist mit diesen drei Winkeladaptern 285 die gesamte Palette von Winkelstellungen erhältlich und einstellbar, wobei auch der Winkeladapter 85 mit den Eingrifföffnungen 286' und 286'' als ein einziges Bauelement eingesetzt werden kann, wobei auch der Winkeladapter 85 mit den Eingrifföffnungen 286' und 286'' als ein einziges Bauelement eingesetzt werden kann, wobei dann dieser Winkeladapter 285 entweder im oberen Bereich oder in seinem unteren B ereich eine eingrifföffnung 286' bzw. 286" aufweist, wodurch die Möglichkeit gegeben ist, durch unterschiedliches Einsetzen dieses Winkeladapters in die fensterartige Durchbrechung 280 beide Winkelbereiche wahlweise zu erfassen, nämlich einmal den Bereich mit dem Winkel von 90° und dem Winkel von 120°, so daß lediglich der in Fig. 24 dargestellte Winkeladapter 285 um 180° verdreht dann in die fensterartige Durchbrechung 280 in dem Schaft 21 des rückwärtigen Schalenteiles 220 eingesetzt wird, um die eingrifföffnung 286' im oberen Bereich zu erhalten, so daß dann bei einem Eingriff des Nockens 287 in die diese Lage einnehmende Eingrifföffnung 286' die Spreizfußstellung erreicht und erhalten wird, so daß allein schon mit einem einzigen Winkeladapter die Winkelstellungen von 90° und 120° erreichbar sind. Hierbei ist jedoch die Anordnung und Zuordnung der Eingrifföffnung in dem Winkeladapter 285 zur gesamten Fläche des Winkeladapters derart, daß bei einem Einsetzen des Winkeladapters 85 nach erfolgter Drehung um 180° auch der für die Spitzfußstellung erforderliche Winkel erreicht wird.

Der plattenförmige Winkeladapter 285 ist beispielsweise vermittels Klemmsitz in der fensterartigen Durchbrechung 280 im Schaft des rückwärtigen Schalenteils 220 gehalten. Entsprechend Fig. 21 bis 23 ist der plattenförmige Winkeladapter 285 an seinem umlaufenden Rand 285a mit einer umlaufenden oder sich über sich gegenüberliegende Bereiche erstreckende Nuten 288 zum Eingriff des die fensterartige Durchbrechung 280 begrenzenden Randes 280a versehen. Bevorzugterweise besteht der plattenförmige Winkeladapter 285 aus einem federnd-elastischen Material, zweckmäßigerweise aus einem Kunststoff und ist dabei mittels Klemmsitz am umlaufenden Rand 80a der fensterartigen Durchbrechung 280 gehalten. Aufgrund der federnd-elastischen Ausgestaltung der Randabschnitte wird der plattenförmige Winkeladapter 285 lediglich in die fensterartige Durchbrechung 280 derart eingedrückt, daß der Winkeladapter 285 im Randbereich der fensterartigen Durchbrechung 280 gehalten ist. Der umlaufende Rand des Winkeladapters 285 und der die fensterartige Durchbrechung 280 begrenzende Rand ist mit Eingriffprofilierungen versehen, d. h. nach Art der Nut- und Feder-Ausgestaltung kann eine Randprofilierung vorgenommen werden.

Nach einer weiteren Ausführungsform gemäß Fig. 22 weist der plattenförmige Winkeladapter 285 an seinen beiden Längsseitenkanten 285b, 285c keilförmig sich nach oben erweiternde Verstärkungen 289, 289a aus einem federnd-elastischen Material auf, so daß der Klemmsitz im Randbereich der fensterartigen Durchbrechung 280 noch erhöht wird. Diese sich keilförmig nach oben erweiternden Verstärkungen 289, 289a an den seitlichen Rändern des plattenförmigen Winkeladapters 285 können auch als bewegbare Leisten ausgebildet sein, die mit einem Mechanismus derart verbunden sind, daß bei einer Betätigung des Mechanismus diese keilförmigen Verstärkungen 289, 289a in den Rand des Winkeladapters 285 eingeschwenkt werden, so daß, wenn der Winkeladapter 285 nicht mittels Klemmsitz in der fensterartigen Durchbrechung 280 gehalten ist, dann nach dieser sogenannten Entriegelung der Winkeladapter 285 aus der fensterartigen Durchbrechung 280 herausgenommen werden kann. Auch andersartig ausgebildete Verriegelungseinrichtungen für die Arretierung und Halterung des Winkeladapters 285 in der fensterartigen Durchbrechung 280 können vorgesehen sein.

Das auswechseln des Winkeladapters 285 kann mittels in der Zeichnung nicht dargestellter zangenartiger Öffnungswerkzeuge erfolgen. Hierzu weist der plattenförmige Winkeladapter 285 in seinem oberen Bereich zwei im Abstand voneinander angeordnete Durchbrechungen 283 auf, in die ein zangenartiges Öffnungswerkzeug einsetzbar ist, so daß bei aufeinanderlaufenden Bewegungsrichtungen in Pfeilrichtung X, X1 der Winkeladapter 285 in diesem Eingriffbereich des Werkzeuges zusammengepreßt wird und somit die entriegelung der leistenartigen Verstärkungen 289, 289a aus dem Randbereich der fensterartigen Durchbrechung 280 erfolgt. Vorausgesetzt ist, daß eine entsprechende federnd-elastische Ausgestaltung des Winkeladapters 285 gegeben ist, wobei jedoch der Winkeladapter 285 im Bereich der Eingrifföffnung 86 für den Nocken 287 eine hohe Eigensteifigkeit aufweisen muß.

Die bevorzugterweise aus Kunststoff bestehenden Wandflächen eines jeden Schalenteiles 220 bzw. 230 sind gitterförmig unter Ausbildung von Durchbrechungen zwischen den sich kreuzenden Gitterstäben, die auch streifen- oder bandförmig ausgebildet sein können, ausgebildet. Diese Ausgestaltung erbringt den Vorteil, daß ein zwischen der Schalenkörperwand und der Extremität angeordnetes Manschettenkisseri insofern lagegesichert und rutschfest angeordnet ist, als die leicht verformbaren Kissenwände in die Durchbrechungen des Schalenkörperwandgitters so gedrückt werden, daß Kissenabschnitte wulstartig aus den Durchbrechungen heraustreten und von den die Durchbrechungen begrenzenden Gitterstäben in ihrer Lage gehalten werden.

Die Eingrifföffnungen 286, 286', 286", 286"' in dem Winkeladapter 85 für den Nocken 287 brauchen nicht als Durchbrechung ausgebildet zu sein; auch nutenförmige Vertiefungen oder Ausnehmungen können in dem Winkeladapter 285 die Lagesicherung und Fixierung des Nockens 287 vornehmen.

Außerdem besteht die Möglichkeit, den Nocken 287 an der Innenwandfläche der Rückwand des rückwärtigen Schalenteiles 220 anzuordnen, der dann in einen entsprechend ausgebildeten und vorangehend beschriebenen Winkeladapter 285 eingreift. Der im rückwärtigen Bereich des Fußteiles 222 des Schalenteiles 220 angeordnet ist.

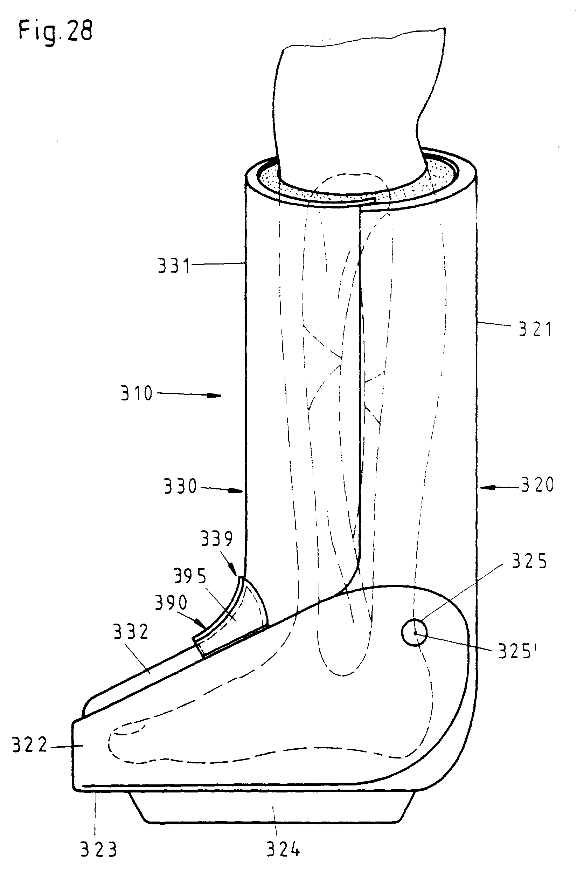

Die in Fig. 28, 29 und 30 dargestellte Vorrichtung zur umschließenden Fixierung von Extremitäten ist als Schalenkörper 310 für den Fuß-/Unterschenkel-Bereich eines Patienten dargestellt. der Schalenkörper 310 besteht aus zwei gegeneinander verspannbaren Schalenteilen, nämlich einem rückwärtigen Schalenteil 320 und einem vorderen Schalenteil 330, wobei beide Schalenteile 320, 330 in etwa eine L-Form aufweisen. Jedes Schalenteil 320 bzw. 330 besteht aus einem Schaft 321 bzw. 331 und einem Fußteil 322 bzw. 332, wobei die Fußteile 322, 332 der beiden Schalenteile mit ihren Schäften 321, 331 über seitliche Schwnkgelenke 325, 335 miteinander verbunden sind, so daß die Fußteile 322, 332 zu den Schäften 321, 331 um Schwenkachsen 325a, 335a verschwenkbar sind. Sowohl die beiden Schalenteile 320, 330 als auch ihre Schwenkgelenke 325, 335 bestehen zweckmäßigerweise aus Kunststoff.

Die Fußteile 322, 332 und die Schäfte 321, 331 der beiden Schalenkörper sind schalenförmig einseitig offen ausgebildet, so daß in dem rückwärtigen Schalenteil 320 eine Aufnahmewanne für den Fuß und den Unterschenkel geschaffen ist. In gleicher Weise ist auch der vordere Schalenkörper 330 ausgebildet, so daß dieser mit seinem Schaft 331 und seinem Fußteil 332 auf den Schaft 321 und das Fußteil 322 des rückwärtigen Schalenteiles 320 aufsetzbar ist. An der Unterseite des Fußteiles 322 des rückwärtigen Schalenteiles 320 ist eine Laufsohle 323 vorgesehen, deren äußere Gestalt an die Abrollbewegung es Fußes beim Gehen angepaßt ist. Hierzu ist die Laufsohle 323 als Gehwiege ausgebildet oder mit einem Gehstollen 324 versehen.

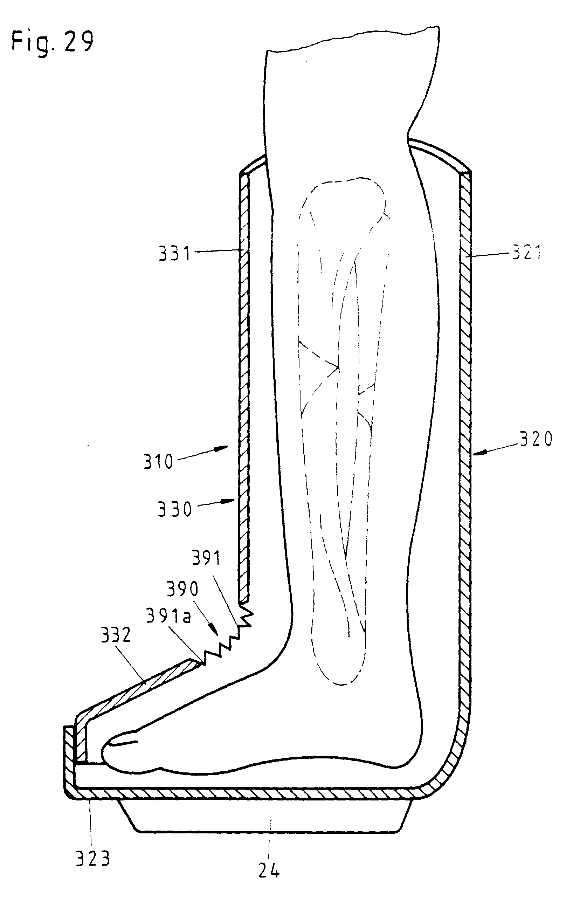

Die Fußteile 322, 332 und die Schäfte 321, 331 der beiden Schalenteile 320, 330 des Schalenkörpers 310 sind in Richtung zum Fuß und zum Unterschenkel schalenförmig ausgebildet, wobei die Ausbildung derart ist, daß das vordere Schalenteil 330 in das rückwärtige Schalenteil 320 derart einsetzbar ist, daß die seitlichen Wandabschnitte des rückwärtigen Schalenteiles 320 die seitlichen Wandabschnitte des Schalenteiles 330 abschnittsweise übergreifen (Fig. 29).

Sowohl das Schalenteil 320 als auch das Schalenteil 330 weisen einen etwa halbkreisförmigen Querschnitt auf, so daß bei aufeinandergesetzten Schalenteilen ein abschnittsweises Übergreifen der Seitenwandabschnitte der beiden Schalenteile 320, 330 erfolgt. Um den Unterschenkel und auch den Fuß seitlich umgreifen zu können, weisen die Schalenteile 320, 330 eine gewisse Elastizität auf, wobei zweckmäßigerweise die Elastizität der Seitenwandungen der beiden Schalenteile 320, 330 von ihren mittigen Bereichen der Schaftteile zu ihren vorderen freien Rändern eines jeden Schalenteiles zunimmt.

Die an den Längsrandbereichen von Fußteil und Schaft zweckmäßigerweise vorgesehenen in der Zeichnung nicht dargestellten Verschlußorgane, die insbesondere aus streifenförmigen Klettverschlußteilen bestehen, sorgen sehr einfach und schnell für den vor dem Evakuieren notwendigen leichten Anpreßdruck des Kissens an den geschlossenen Schalenkörper. Auf diese Weise läßt sich eine Hülse absolut korrekter Paßform erzeugen und zunächst über den Klettverschluß festlegen. Nach erfolgter Evakuierung liegt dann eine Hülse vor, die in sich verfestigt und in ihrer Form stabilisiert ist und die Extremität lagegesichert hält.

Eine Bewegung im Fußgelenk des Patienten kann verhindert werden, indem der Neigungswinkel des Schaftteils bezüglich des Fußteiles um die Schwenkachse 325 bzw. 335 fest eingestellt wird. Eine Wahl dieses fest eingestellten Winkels kann in Abhängigkeit von der zu behandelnden Verletzung innerhalb des gesamten einstellbaren Schwenkwinkelbereiches erfolgen. Das Feststellen auf einen bestimmten Winkel erfolgt mittels entsprechend ausgebildeter Verstelleinrichtungen, wobei bei dem rückwärtigen Schaftteil 320 eine Winkelvorgabe zur Einstellung eines Winkels von 90° oder von 120° zwischen dem Fußteil 322 und dem Schaft 321 vermittels eines in der Zeichnung nicht dargestellten Winkeladapters erfolgen kann.

Um eine Anpassung des vorderen Schalenteiles 330 an die Winkelstellung des Fußteiles 322 zum Schaft 321 des rückwärtigen Schalenteils 320 vornehmen zu können, ist das vordere Schalenteil 330 mit einer Winkeleinstelleinrichtung 390 versehen, vermittels der das Fußteil 332 zum Schaft 331 in einer vorgegebenen Winkelstellung arretierbar ist, wobei diese Wikelstellung mit derjenigen Winkelstellung des Fußteiles 322 zum Schaft 321 des rückwärtigen Schalenteiles 320 übereinstimmt.

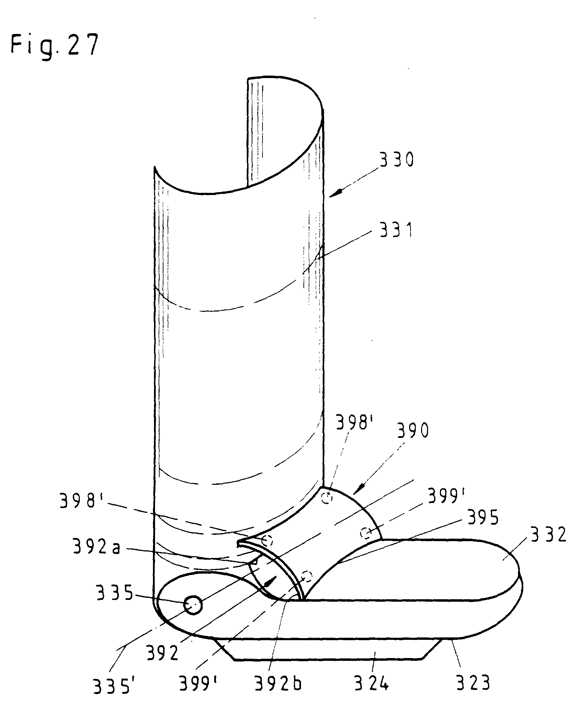

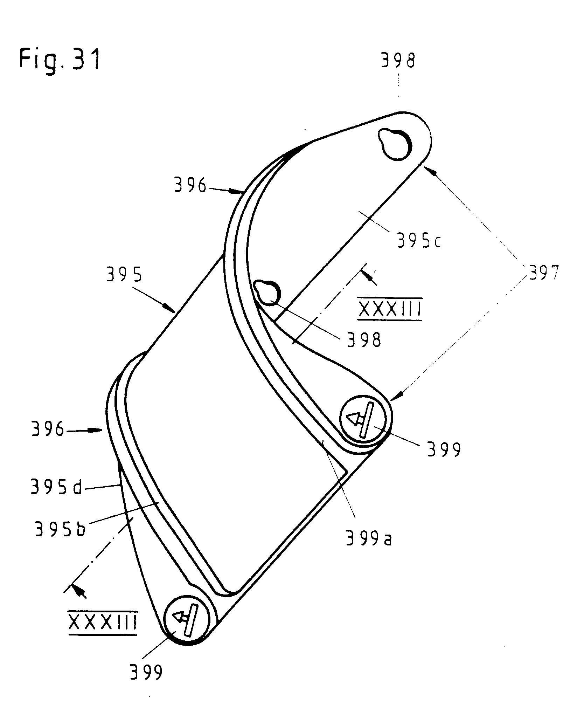

Diese Winkelstelleinrichtung 390 besteht aus einem zwischen dem Fußteil 332 und dem Schaft 331 des vorderen Schalenkörpers 330 angeordneten, dem Außenprofil des Schalenkörpers 310 bzw. des vorderen Schalenteils 330 entsprechend geformten, plattenförmigen Winkeladapter 395, der mittels Rast- und Verriegelungseinrichtungen 397 an dem Schaft 331 und dem Fußteil 332 des vorderen Schalenteils 330 lösbar gehalten ist (Fig. 27 und 31). Dieser Winkeladapter 395 übergreift laschenförmig vorderseitig den Verbindungsbereich 339 zwischen dem Fußteil 332 und dem Schaft 331 des vorderen Schalenkörpers 330.

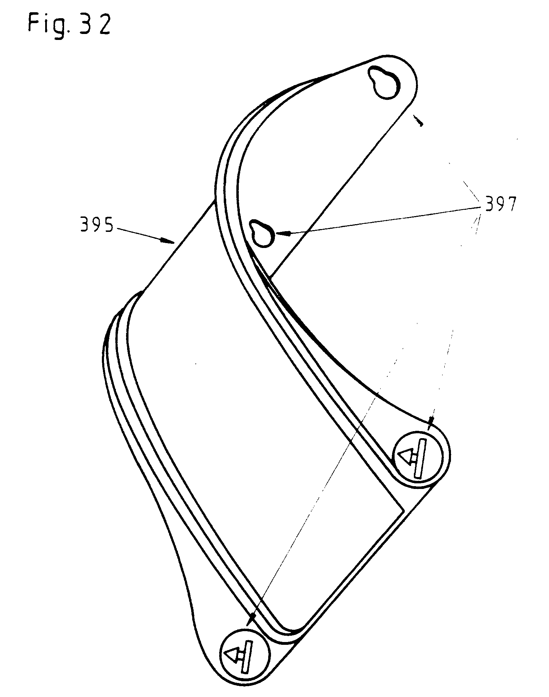

Dieser plattenförmige Winkeladapter 335 weist eine Breite auf, aufgrund der das Fußteil 332 zu dem Schaft 331 des Schalenteils 330 eine Winkelstellung von 90° einnimmt (Fig. 31). Bei dieser Winkelstellung handelt es sich um die Normalstellung zwischen dem Fuß und Unterschenkel. Eine weitere Ausführungsform des Winkeladapters 395 sieht eine Breite vor, aufgrund der das Fußteil 332 zu dem Schaft 331 des Schalenteils 330 eine Winkelstellung von 120° einnimmt, so daß, wenn diese Ausführungsform des Winkeladapters zwischen dem Fußteil 332 und dem Schaft 331 des Schalenteils 330 eingesetzt wird, das Fußteil zu dem Schaft 331 die Spitzfußwinkelstellung erhalten wird (Fig. 32).

Das Fußteil 332 und der Schaft 331 des vorderen Schalenteils 330 weist im Verbindungsbereich 339 dieser beiden Teile eine fensterartige Durchbrechung 392 auf, die sich bis etwa in den Seitenbereich des Fußteiles 332 und des Schaftes 331 erstreckt, so daß der an dem Schalenteil 330 angebrachte Winkeladapter 395 diese fensterartige Durchbrechung 392 in Form einer Abdecklasche übergreift, wobei die Abmessungen des Winkeladapters 395 derart bemessen sind, daß dieser in die fensterartige Durchbrechung 332 einsetzbar bzw. mit seinem umlaufenden Rand auf den die fensterartige Durchbrechung 392 begrenzenden Rand aufsetzbar und hier mittels Klemm- bzw. Preßsitz gehalten ist.

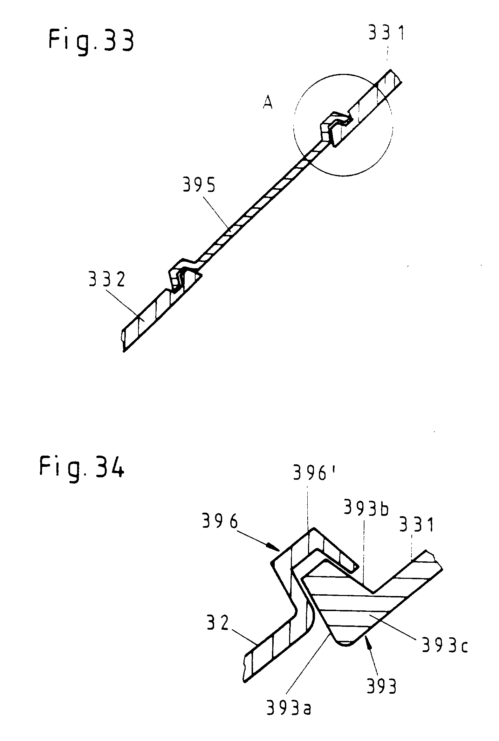

Zur Halterung des Winkeladapters 395 an den Wandflächen des Fußteiles 332 und des Schaftes 331 des Schalenteils 330 weisen die gegenüberliegenden, quer zur Schaftlängsrichtung verlaufenden Ränder 392a, 392b der fensterartigen Durchbrechung 392 außenseitig wulstartig oder andersartig profilierte leistenförmige Verstärkungen 393 auf, während der Winkeladapter 395 an seinem oberen Rand 395a und an seinem unteren Rand 395b mit den Verstärkungen 393 an den Rändern 392a, 392b entsprechend ausgebildeten Gegenprofilen als Randprofile 96 versehen sind, so daß der Winkeladapter 395 mittels Klemmsitz an den Verstärkungen 393 an dem Fußteil 332 oder dem Schaft 331 bzw. an diesem des vorderen Schalenteils 330 gehalten ist (Fig. 33).

Die Befestigung und Halterung des Winkeladapters 395 an dem Schaft 331 und dem Fußteil 332 des vorderen Schalenteils 30 erfolgt entsprechend Fig. 31 vermittels zweier an dem einen Ende 395c des Winkeladapters, und zwar innenwandseitig feststehend angeordneten Verriegelungshaken 398, die in Durchbrechungen 398' in den Wandflächen des Schaftes 331 und des Fußteiles 332 des Schalenteils 330 eingreifen. Mit diesem seinem Ende 395c wird der Winkeladapter 395 in die vorgesehenen Durchbrechungen 398' in den Wandflächen des Schaftes 331 und des Fußteiles 332 des Schalenteils 330 eingehakt. An seinem anderen Ende 395d trägt der Winkeladapter 395 zwei drehbare, als Exzenter ausgebildete Verriegelungshaken 399, die ebenfalls in entsprechende Durchbrechungen 399' in den Wandflächen des Schaftes 331 und des Fußteiles 32 des Schalenteils 330 eingreifen, wobei die Verriegelung durch Verdrehen der in diese Durchbrechungen 399' eingeführten Verriegelungshaken 399 erfolgt, so daß durch diese drehbaren und als Exzenter ausgebildeten Verriegelungshaken 399 der Winkeladapter 395 über die Gegenteile, hier Fußteil 332 und Schaft 331, gespannt, so daß dadurch ein spielfreier Preßsitz, insbesondere an den schrägen Flächen der wulstartigen Randverstärkungen 393 erreicht wird (Fig. 34). Die Anzahl der feststehenden Verriegelungshaken 398 und der verdrehbaren Verriegelungshaken 399 kann beliebig gewählt sein. Wesentlich ist, daß jeweils ein feststehender Verriegelungshaken 398 und ein verdrehbarer Verriegelungshaken 399 miteinander korrespondieren und in die Durchbrechungen 399' im Fußteil 332 des Schalenteils 330 eingreifen.

An Stelle von Verriegelungshaken 398, 399 können jedoch auch andersartig ausgestaltete Befestigungsmittel eingesetzt werden.

Der plattenförmige Winkeladapter 395 besteht bevorzugterweise aus einem federnd-elastischen Material, zweckmäßigerweise aus einem Kunststoff.

Auch die beiden Schalenteile 320, 330 bestehen aus einem Kunststoff.

Die bevorzugterweise aus Kunstoff bestehenden Wandflächen eines jeden Schalenteils 320 bzw. 330 sind gitterförmig unter Ausbildung von Durchbrechungen zwischen den sich kreuzenden Gitterstäben, die auch streifen- oder bandförmig ausgebildet sein könnn, ausgebildet. Diese Ausgestaltung erbringt den Vorteil, daß ein zwischen der Schalenkörperwand und der Extremität angeordnetes Manschettenkisseninsofern lagegesichert und rutschfest angeordnet ist, als die leicht verformbaren Kissenwände in die Durchbrechungen des Schalenkörperwandgitters so gedrückt werden, daß Kissenabschnitte wulstartig aus den Durchbrechungen heraustreten bzw. in diesen zu liegen kommen und somit von den die Durchbrechungen begrenzenden Gitterstäben in ihrer Lage gehalten werden.

Eine weitere Möglichkeit der Winkelverstellbarkeit des Fußteiles 332 zum Schaft 331 des vorderen Schalenteils 330 besteht gemäß Fig. 29 darin, daß das zum Schaft 331 verstellbare Fußteil 332 des vorderen Schalenteiles 330 über einen quer zur Schaftlängsrichtung verlaufenden ziehharmonikabalgartig ausgebildeten Abschnitt 391 mit einer Anzahl von gefalteten und filmscharnierartig miteinander verbundenen Materialstreifen 391a verbunden ist, wobei die das Fußteil 332 und den Schaft 331 miteinander verbindenden, seitlich an dem Schalenkörper 310 ausgebildeten Schwenkgelenke 335 mit Winkelarretierungseinrichtungen versehen sind. Bei dieser Ausführungsform ist der der sonst mittels des plattenförmigen Winkeladapters 395 verschlossene fensterartige Durchbrechung 392 zwischen dem Fußteil 332 und dem Schaft 331 entsprechende Bereich über einen Abschnitt 391 ausgefüllt, der aus dem gleichen Material besteht, aus dem auch der Schalenteil 330 gefertigt ist. Dieser Abschnitt 391 ist balgartig entsprechend einer Zieharmonika ausgebildet, wobei die einzelnen Materialstreifen 391a filmscharnierartig verbunden sind, so daß in Abhängigkeit von der jeweiligen Winkelstellung des Fußteiles 332 zu dem Schaft 331 des chalenteils 330 sich dieser dehnbare Abschnitt 391 anpaßt. Die im Bereich der Schwenkgelenke 335 vorgesehenen Winkelarretierungseinrichtungen sind in an sich bekannter Weise ausgebildet; sie bestehen aus Feststelleinrichtungen, z. B. Feststellschrauben oder dgl., so daß die vorgegebene Winkelstellung beibehalten und nur nach Lösen der Winkelarretierungseinrichtung verändert werden kann.

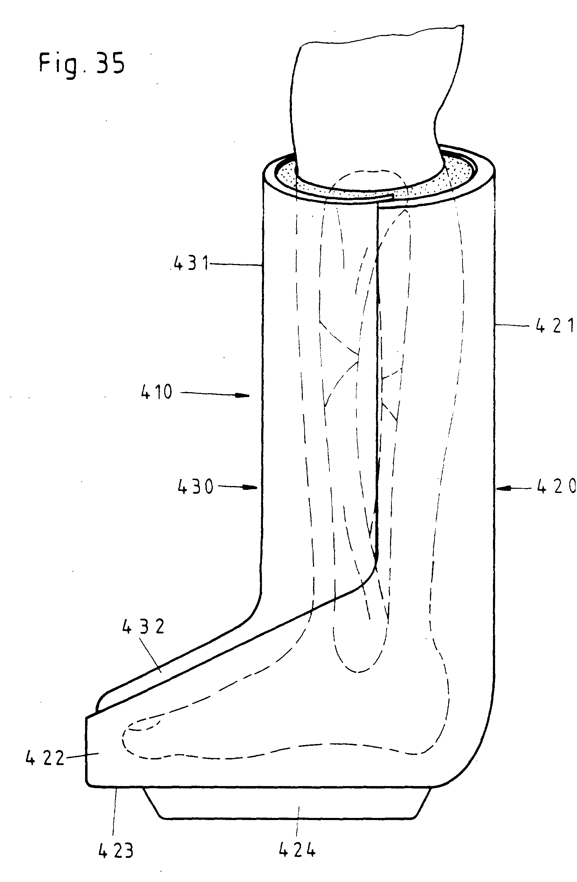

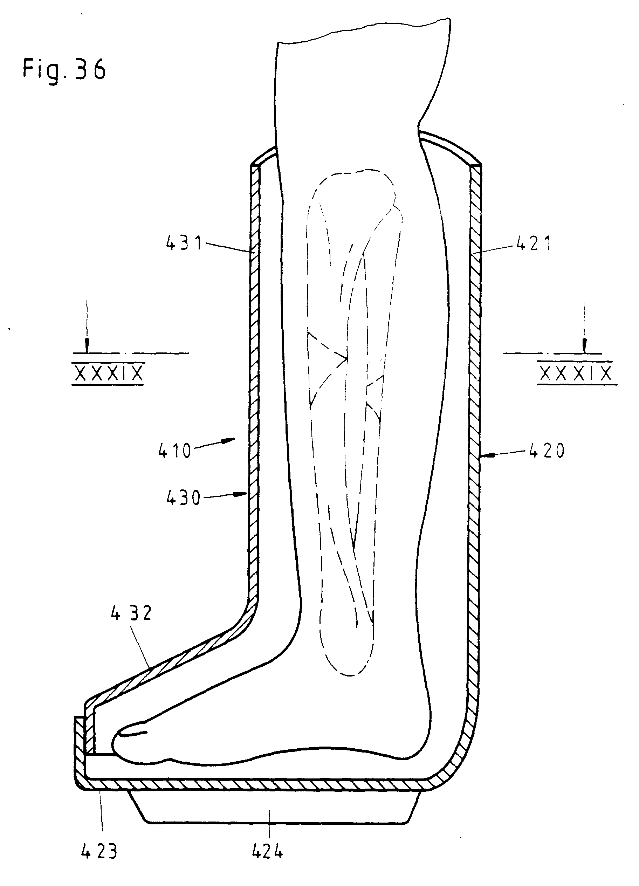

Die Vorrichtung nach Fig. 35 und 36 ist als Schalenkörper für den Fuß-/Unterschenkel-Bereich eines Patienten ausgebildet.

Der Schalenkörper 410 besteht aus zwei gegeneinander verspannbaren Schalenteilen, nämlich einem rückwärtigen Schalenteil 420 und einem vorderen Schalenteil 430, wobei beide Schalenteile 420, 430 in etwa eine L-Form aufweisen. Jedes Schalenteil 420 bzw. 430 besteht aus einem Schaft 421 bzw. 431 und einem Fußteil 422 bzw. 432, wobei das Fußteil 422 des Schalenteiles 420 mit seinem Schaft 421 über in den Abbildungen nicht dargestellte seitliche Schwenkgelenke 35 miteinander verbunden ist. Sowohl die beiden Schalenteile 420, 430 als auch die Gelenke bestehen zweckmäjßigerweise aus Kunststoff.

Das Fußteil 422 und der Schaft 421 des rückwärtigen Schalenteils 420 sind schalenförmig nach oben und nach vorn offen ausgebildet, so daß eine Aufnahmewanne für den Fuß und den Unterschenkel geschaffen ist. An der Unterseite des Fußteils 422 ist eine Laufsohle 423 vorgesehen, deren äußere Gestalt an die Abrollbewegung des Fußes beim Gehen angepaßt ist. Hierzu ist die Laufsohle 423 als Gehwiege ausgebildet oder mit einem Gehstollen 424 versehen.

Das Fußteil 432 und der Schaft 431 des vorderen Schalenteiles 430 des Schalenkörpers 410 sind in Richtung zum Fuß und zum Unterschenkel schalenförmig ausgebildet, wobei dieses Schalenteil 430 so ausgebildet ist, daß das vordere Schalenteil 430 in das rückwärtige Schalenteil 420 derart einsetzbar ist, daß die seitlichen Wandabschnitte des Schalenteiles 420 die seitlichen Wandabschnitte des Schalenteiles 430 abschnittsweise übergreifen.

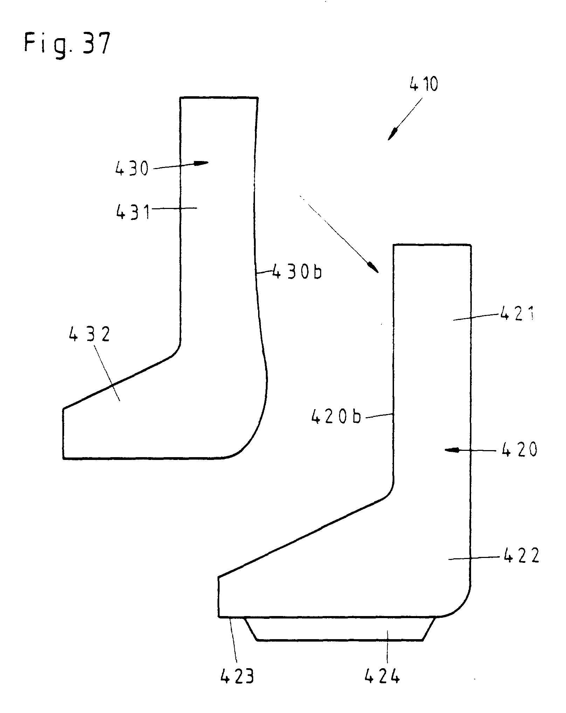

Sowohl das Schalenteil 420 als auch das Schalenteil 430 weisen einen etwa halbkreisförmigen Querschnitt auf, so daß bei ineinandergesetzten Schalenteilen ein abschnittsweises Übergreifen der Seitenwandabschnitte der beiden Schalenteile 420, 430 erfolgt (Fig. 37, 38 und 39). Um den Unterschenkel und auch den Fuß seitlich umgreifen zu können, weisen die Schalenteile 420, 430 eine gewisse Elastizität auf, wobei zweckmäßigerweise die Elastizität der Seitenwandungen der beiden Schalenteile 420, 430 von ihren mittigen Bereichen der Schalenteile zu ihren vorderen freien Rändern 420b bzw. 430b eines jeden Schalenteiles zunimmt.

Zwischen den beiden gegeneinander verspannbaren Schalenteilen 420, 430 und der Extremität ist ebenfalls ein in der Zeichnung nicht dargestelltes verformbares, vakuumdicht ausgebildetes und mit zumindest einem Ventil versehenes Kissen vorgesehen, das aus einer Blase besteht, in der eine Vielzahl von relativ zueinander beweglichen Füllkörpern vorgesehen ist. Das eng und passend an dem zu behandelnden Gliedabschnitt anlegbare Kissen wird bei einer innerhalb kurzer Zeit durchführbaren Evakuierung in der während des Anlegevorganges geschaffenen Form hart und formstabil, so daß in Verbindung mit den harten Schalenteilen eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entsteht und an der Innenseite auch keine Kanten oder Vorsprünge gebildet werden können. Anstelle eines derartigen Manschettenkissens kann auch ein anderweitiges Polster, Strumpf oder dgl. zur Zwischenraumausfüllung eingesetzt werden.

Die an den Längsrandbereichen von Fußteil und Schaft zweckmäßigerweise vorgesehenen Verschlußorgane, die insbesondere aus streifenförmigen Klettverschlußteilen bestehen, sorgen sehr einfach und schnell für den vor dem Evakuieren notwendigen leichten Anpreßdruck des Kissens an den geschlossenen Schalenkörper. Auf diese Weise läßt sich eine Hülse absolut korrekter Paßform erzeugen und zunächst über den Klettverschluß festlegen. Nach erfolgter Evakuierung liegt dann eine Hülse vor, die in sich verfestigt, in ihrer Form stabilisiert ist und die Extremität lagegesichert hält.

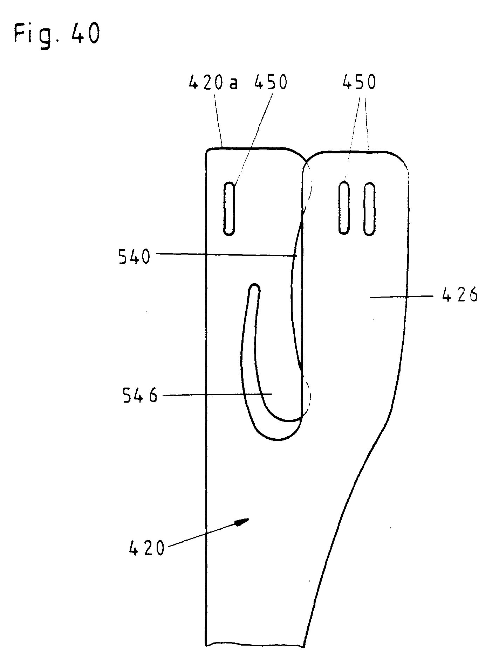

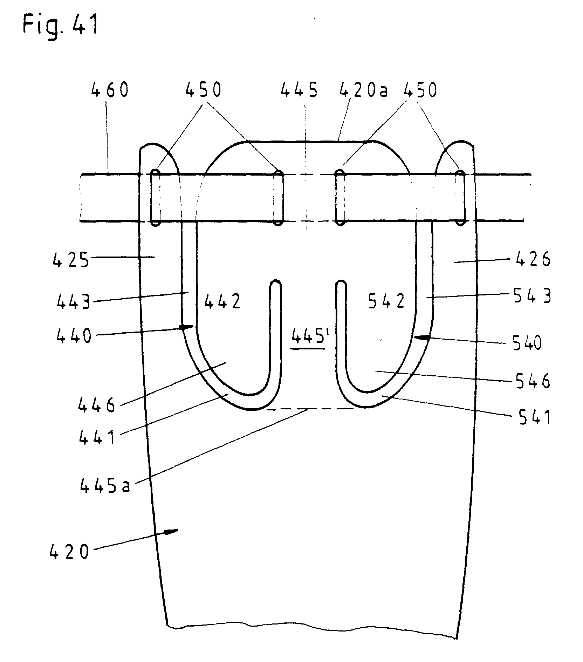

Um den Schalenkörper 410 den jeweiligen vorliegenden Wadengröjßen und -formen des Patienten anpassen zu können, ist das rückwärtige Schalenteil 420 mit einer Anzahl von seinem oberen umlaufenden Rand 20a ausgehenden schlitzförmigen Durchbrechungen 440, 540 versehen, die sich in Schaftlängsrichtung des Schalenteiles 420 erstrecken. Bei dem in Fig. 40 und 41 dargestellten Ausführungsbeispiel sind in der rückwärtigen Wand des Schalenteiles 420 zwei schlitzförmige Durchbrechungen 440, 540 vorgesehen, die parallel zueinander verlaufend sind und durch die mindestens ein abbiegbarer Abschnitt 445 gebildet wird. Die beiden schlitzförmigen Durchbrechungen 440, 540 erstrecken sich in Schaftlängsrichtung des Schalenteiles 420 derart, daß in dem rückwärtigen Schalenteil 20 Wandabschnitte 425, 426, 445 ausgebildet werden, wobei diese Wandabschnitte 425, 426, 445 mit einer Einrichtung 460 auch auf die jeweils erforderliche Stellung fixierbar sein können (Fig. 41). Die Wandabschnitte 425, 426 sind feststehend und wirken stabilisierend.

Bei dem in Fig. 40 und 41 dargestellten Ausführungsbeispiel sind die beiden schlitzförmigen Durchbrechungen 440, 540 U-förmig verlaufend ausgebildet. Die U-förmigen Durchbrechungsabschnitte sind bei 441, 541 angedeutet. Jede schlitzförmige Durchbrechung 440, 540 geht vom oberen Rand 420a der rückwärtigen Seitenwandung des rückwärtigen Schalenteiles 420 aus und verläuft zunächst mit einem Abschnitt 443 in Längsrichtung des Schaftes 421 des Schalenteiles 420, wobei dann dieser Abschnitt 443 in den U-förmigen Abschnitt 441 übergeht, dessen auslaufender Schenkelabschnitt 442 in etwa parallel zu dem Durchbrechungsabschnitt 443 verlaufend ist, so wie dies in Fig. 41 dargestellt ist. Die schlitzförmige Durchbrechung 540 ist entsprechend der schlitzförmigen Durchbrechung 440 ausgebildet und wird ebenfalls von einem in Schaftlängsrichtung verlaufenden Abschnitt 543 gebildet, an den sich der U-förmige Abschnitt 541 anschließt, dessen Schenkelabschnitt 542 parallel zu dem Durchbrechungsabschnitt 143 verlaufend ist, so daß die beiden innen liegenden Schenkelabschnitte 442, 542 der beiden Durchbrechungen 440, 540 einander zugekehrt sind. Aufgrund dieser Anordnung und Ausbildung von schlitzförmigen Durchbrechungen 440, 540 in der rückwärtigen Wand des Schalenteiles 20 werden lappenförmige Randabschnitte 446, 546 ausgebildet, die die Wirkung von federnd-elastischen Zungen aufweisen und die es möglich machen, daß die die schlitzförmigen Durchbrechungen 440, 540 seitlich begrenzenden Wandabschnitte 425, 426 des rückwärtigen Schalenteiles 420 sich gegen die federnden Abschnitte 446, 546 derart verschieben lassen, daß eine Anpassung des Schalenteiles 420 und somit des Schalenkörpers 410 an die Wade des Patienten möglich ist. Diese Verschiebbarkeit der federnd-elastischen Wandabschnitte des rückwärtigen Schalenteiles 420 ist in Fig. 40 dargestellt.

Die Anordnung der beiden schlitzförmigen Durchbrechungen 440, 540 erfolgt in demjenigen Bereich des rückwärtigen Schalenteiles 420 des Schalenkörpers 410, der die Wade des Unterschenkels des Patienten erfaßt.

Der abbiegbare Abschnitt 445 in der Wand des rückwärtigen Schalenteiles 420 ist mit dessen Wand über einen Steg 445' verbunden, der federnd-elastisch an der Wand angeformt und gehalten ist oder über eine Gelenkverbindung mit der Wand verbunden ist, so daß im letzteren Fall der Abschnitt 445 die Funktion einer Wippe hat und sich der Wade und den Wadenmuskelbewegungen anpaßt. Im Falle der Verwendung einer Gelenkverbindung erfolgt eine Druckentlastung der Wade, da der Abschnitt 445 quasi freischwingend, doch immer in Anlage an die Wade ist. Vermittels der Gelenkverbindung, die auch als Filmscharnier ausgebildet sein kann, ist der Abschnitt 445 zusammen mit seinem Steg 445' um die Schwenkachse 445a bewegbar (Fig. 41).

Um den rückwärtigen Schalenteil 420 des Schalenkörpers 410 nach erfolgter Anpassung an die Wade des Patienten in der angepaßten Stellung arretieren zu können, weisen die federnden und einzeln ausgebildeten Abschnitte 425, 426, 445 des Schalenteiles 420 benachbart zum oberen umlaufenden Rand 420 a eine Anzahl von schlitzförmigen Durchbrechungen 450 zur Aufnahme einer streifenförmigen Verschlußeinrichtung 460, die als Gurtband ausgebildet ist oder aus streifenförmigen Klettverschlußteilen bestehen kann, um in der Anpaßstellung das rückwärtige Schalenteil 420 im Bereich seines Schaftes 421 feststellen zu können. Wird der rückwärtige Schalenteil 420 ohne einen vorderen Schalenteil 430 verwendet, dann greifen die Verschlußorgane ausschließlich an den feststehenden Wandabschnitten 425, 425 an. An diesen können die Verschlußorgane auch einendseitig befestigt sein. Diese Art der Anbringung der Verschlußorgane ist jedoch in allen Fällen vorzuziehen, da die freie Beweglichkeit der Abschnitte 445 vermittels dessen Steg 445' nicht beeinträchtigt wird, wobei auch eine Längsverschieblichkeit des Steges nach außen möglich ist.

Die so ausgebildete Fixierungsvorrichtung ist somit mit einer integrierten Wadenanpassung versehen, ohne daß dabei die Formstabilität des Schalenkörpers 410 beeinträchtigt wird. Trotz der hohen Eigensteifigkeit insbesondere des rückwärtigen Schalenteiles 420 ist eine Anpassungselastizität an den Unterschenkel und die Wade des Patienten gegeben. Durch den in den rückwärtigen Schalenteil 420 eingesetzten vorderen Schalenteil 430 wird eine Bewegung im rückwärtigen Schalenteil 420 verhindert. Die Normalwinkelstellung zwischen Fuß und Unterschenkel von 90° ist somit immer einhaltbar. Der Unterschenkel liegt bei stationärer Behandlung des Patienten nach einer Operation in dem rückwärtigen Schalenteil 420 und ist in diesem fixiert, so daß die korrekte anatomische Stellung eingehalten werden kann. Trotz der Fixierung des Fußes und des Unterschenkels in der Normalwinkelstellung von 90° wird eine hohe Stabilisierung erreicht.

Um eine Anpassungsfähigkeit des rückwärtigen Schaftteiles 420 an die Wade zu erreichen, kann die Rückwand und/oder die Seitenwände des Schaftteiles 420 auch andersartig ausgebildet sein und verlaufende Einschnitte als wie die vorangehend beschriebenen schlitzförmigen Durchbrechungen 440, 540 aufweisen. Da das Schalenteil 420 aus Kunststoff besteht, ist die MÖglichkeit gegeben, an Stelle von schlitzartigen Durchbrechungen 440, 540 in Schalenteillängsrichtung verlaufende Abschnitte in Form von Materialverdünnungen vorzusehen, die sich ziehharmonikaartig bei einem Verschieben der einzelnen Wandabschnitte gegeneinander verformen.

## Patentansprüche

1. Vorrichtung zur umschließenden Fixierung von Extremitäten und Extremitätenbereichen im Bereich von Unterschenkel und Oberschenkel, bestehend aus einem Schalenkörper (10) aus zwei gegeneinander verspannbaren L-förmigen Schalenteilen (20,30) mit einem etwa halbkreisförmigen Querschnittsprofil, die die zu umschließende Extremität umgeben, wobei zwischen den Schalenteilen (20,30) und der Extremität mindestens ein evakuierbares Manschettenkissen vorgesehen ist, in dessen zwischen den beiden Kissenwänden gelegenen Innenraum eine Vielzahl von relativ zueinander beweglichen Füllkörpern vorgesehen ist,dadurch gekennzeichnet, daß die beiden Schalenteile (20,30) eine sich über die gesamte Wandfläche eines jeden Schalenteils erstreckende Gitterstruktur aufweisen, die aus Gitterstreben und/oder Gitterrippen in Längs- (11) und Querrippen (12) besteht,wobei zumindest eine Längsrippe (11a) pro Schalenteil vorgesehen ist, die sich im wesentlichen über die gesamte Länge des Schalenteils erstreckt und die mindestens so breit oder mit einem größeren Querschnitt ausgestaltet ist wie die übrigen Längsstreben, Querstreben oder -rippen (11,12) und die Anschlußstellen (13) der Querrippen (12) an der sich über die gesamte Länge erstreckenden Längsrippe (11a) breiter als der sich anschließende, vorzugsweise in der Breite oder im Durchmesser verjüngend ausgebildete Querrippenteil sind,daß zumindest der rückseitige Schaftoberteil (21) des Schalenkörpers (10) einen Längsschlitz (14) zur Breiten- oder Durchmesserverstellung aufweist, wobei die den Längsschlitz (14) begrenzenden Längsrippen über ein lösbares Verbindungselement (15;115) auf den gewünschten Abstand einstellbar und fixierbar sind, und daß das vordere Schalenteil (30) ein zu dessen Schaft (31) über ein Gelenk (35) verschwenkbares Fußteil (32) und das rückseitige Schalenteil (20) ein zu seinem Schaft (21) über ein zweites Gelenk (25) verschwenkbares Fußteil (22) aufweist, wobei das Gelenk (35) und/oder das zweite Gelenk (25) vermittels einer Winkeleinstelleinrichtung (90) in jeder Winkelstellung arretierbar ist/sind.

2. Vorrichtung nach Anspruch 1, dadurch gekennzeichnet, daß die Gitterstreben und/oder Gitterrippen (11,12) eine Breite oder einen Durchmesser zwischen 1,5 cm und 3 cm haben.

3. Vorrichtung nach einem der Ansprüche 1 und 2, dadurch gekennzeichnet, daß die Gitterstreben und/oder Gitterrippen (11,12) jeweils in Abhängigkeit von den auf sie wirkenden Belastungen unterschiedlich breit und/oder mit einem unterschiedlichen Durchmesser versehen sind.

4. Vorrichtung nach einem der Ansprüche 1 bis 3, dadurch gekennzeichnet, daß das zum Schaft (31) verstellbare Fußteil (32) des vorderen Schalenteiles (30) über einen quer zur Schaftlängsrichtung verlaufenden ziehharmonikabalgartig ausgebildeten Abschnitt (91) mit einer Anzahl von gefalteten, filmscharnierartig miteinander verbundenen Materialstreifen (91a) verbunden ist, wobei die das Fußteil (32) und den Schaft (31) miteinander verbindenden, seitlich an dem Schalenkörper (10) ausgebildeten Schwenkgelenke (35) mit Winkelarretierungseinrichtungen versehen sind.

5. Vorrichtung nach einem der Ansprüche 1 bis 3, dadurch gekennzeichnet, daß die Winkeleinstelleinrichtung (90) aus einem zwischen dem Fußteil (32) und dem Schaft (31) des vorderen Schalenteils (30) angeordneten, dem Außenprofil des Schalenkörpers (10) bzw. des vorderen Schalenteils (30) entsprechend geformten rippen-, gitter- oder plattenförmigen Winkeladapter (95) besteht, der mittels Rast- und Verriegelungseinrichtungen (97) an dem Schaft (31) und dem Fußteil (32) des vorderen Schalenteils (30) lösbar gehalten ist.

6. Vorrichtung nach Anspruch 5, dadurch gekennzeichnet, daß der rippen-, gitter- oder plattenförmige Winkeladapter (95) eine Breite aufweist, aufgrund der das Fußteil (32) zu dem Schaft (31) des vorderen Schalenteils (30) eine Winkelstellung von 90° einnimmt.

7. Vorrichtung nach Anspruch 5, dadurch gekennzeichnet, daß der rippen-, gitter- oder plattenförmige Winkeladapter (95) eine Breite aufweist, aufgrund der das Fußteil (32) zu dem Schaft (31) des vorderen Schalenteils (30) eine Winkelstellung von 120° einnimmt.

8. Vorrichtung nach einem der Ansprüche 1 bis 7, dadurch gekennzeichnet, daß das Fußteil (32) und der Schaft (31) des vorderen Schalenteils (30) im Verbindungsbereich (39) eine fensterartige Durchbrechung (92) aufweist, die sich bis in den Seitenbereich des Fußteiles (32) und des Schaftes (31) erstreckt und in der der rippen-, gitter- oder plattenförmige Winkeladapter (95) angeordnet ist.

9. Vorrichtung nach Anspruch 8, dadurch gekennzeichnet, daß die gegenüberliegenden, quer zur Schaftlängsrichtung verlaufenden Ränder (92a,92b) der fensterartigen Durchbrechung (92) außenseitig wulstartige oder andersartig profilierte, leistenförmige Verstärkungen (93) aufweisen und daß der rippen-, gitter- oder plattenförmige Winkeladapter (95) an seinem oberen (95a) und unteren Rand (95b) mit den Verstärkungen (93) entsprechend ausgebildeten Gegenprofilen als Randprofile (96) versehen und mittels Klemmsitz an den Verstärkungen (93) an dem Fußteil (32) oder dem Schaft (31) des vorderen Schalenteils (30) gehalten ist.

10. Vorrichtung nach Anspruch 9, dadurch gekennzeichnet, daß die Randverstärkungen (93) an dem Fußteil (32) und dem Schaft (31) des vorderen Schalenteils (30) ein Querschnittsprofil mit einer quadratischen oder rechteckförmigen, konisch nach oben verlaufende Seitenflächen (93a,93b) aufweisende Fläche (93c) aufweisen, und daß die Randprofile (96) an dem rippen-, gitter- oder plattenförmigen Winkeladapter (95) als entsprechendes, die Randverstärkungen (93) an dem Fußteil (32) und dem Schaft (31) übergreifendes Gegenprofil (96') ausgebildet sind.

11. Vorrichtung nach einem der Ansprüche 5 bis 10, dadurch gekennzeichnet, daß zur Befestigung des rippen-, gitter- oder plattenförmigen Winkeladapters (95) an dem Schaft (31) und dem Fußteil (32) des vorderen Schalenteils (30) der Winkeladapter (95) einendseitig (95c) und innenwandseitig mindestens zwei feststehende Verriegelungshaken (98), die in Durchbrechungen (98') in den Wandflächen des Schaftes (31) und des Fußteils (32) eingreifen, und an seinem anderen Ende (95d) mindestens zwei drehbare, als Exzenter ausgebildete Verriegelungshaken (99) aufweist, die in entsprechende Durchbrechungen (99') in den Wandflächen des Schaftes (31) und des Fußteiles (32) des vorderen Schalenteils (30) eingreifen.

12. Vorrichtung nach einem der Ansprüche 5 bis 11, dadurch gekennzeichnet, daß der rippen-, gitter- oder plattenförmige Winkeladapter (95) aus einem federnd-elastischen Material, zweckmäßigerweise aus einem Kunststoff besteht.

13. Vorrichtung nach einem der Ansprüche 1 bis 12, dadurch gekennzeichnet, daß die beiden Schalenteile (20,30) und/oder die Winkeleinstelleinrichtung (90) aus einem Kunststoff bestehen.

14. Vorrichtung nach einem der Ansprüche 1 bis 13, dadurch gekennzeichnet, daß an den Schalenteileninnenseiten ein Manschettenkissen oder Polster angeordnet ist, in dem eine Vielzahl von relativ zueinander beweglichen Füllkörpern enthalten ist.

15. Vorrichtung nach einem der Ansprüche 1 bis 14, dadurch gekennzeichnet, daß den Längsschlitz (14) umrahmende Führungen (16',17') über ein lösbares Verbindungselement (15;115) auf den gewünschten Abstand einstellbar und fixierbar sind, wobei das Verbindungselement ein Schieber (15;115) mit die Führungen teilweise im Umfang umgreifenden oder in die Führung eingreifenden Randprofilierungen ist.

16. Vorrichtung nach Anspruch 15, dadurch gekennzeichnet, daß eine der beiden Führungen (16',17') die andere in Richtung der Schalenoberteillängsachse um eine Länge (a) überragt, die mindestens so groß ist wie die Länge (1) des Schiebers oder des Verbindungselementes, wobei eine der beiden Führungen (16') im wesentlichen bis zum Schalenoberteilrand (18) ausgebildet ist.

17. Vorrichtung nach Anspruch 15, dadurch gekennzeichnet, daß das lösbare Verbindungselement oder der Schieber (15;115) mittels eines Anschlages begrenzt in Richtung des Schaftoberteilrandes (18;118) verschiebbar ist.

18. Vorrichtung nach einem der Ansprüche 1 bis 17, dadurch gekennzeichnet, daß das Schaftoberteil (10) zwei im wesentlichen parallel laufende Rippen oder wulstförmige Erhebungen (16,17) aufweist, die in entsprechend geformte Nuten des Schiebers eingreifen, der längsverschiebbar, aber gegen ein seitliches Herausnehmen gesichert ist.

19. Vorrichtung nach Anspruch 18, dadurch gekennzeichnet, daß die führungen (16',17') eine Schwalbenschwanzführung ist.

20. Vorrichtung nach einem der Ansprüche 1 bis 19, dadurch gekennzeichnet, daß das Verbindungselement oder der Schieber (15;115) über zwei Längsrippen des Schaftoberteils greift und entlang diesem führbar ist.

21. Vorrichtung nach einem der Ansprüche 1 bis 20, dadurch gekennzeichnet, daß die beabstandeten, einen Zwischenraum bildenden Längsrippen mit wulstartigen, Führungsschienen (16',17') bildenden Erhebungen (16,17) versehen sind.

22. Vorrichtung nach einem der Ansprüche 1 bis 21, dadurch gekennzeichnet, daß von den beiden, eine etwa L-Form aufweisenden Schalenteilen (220;230) mit einem etwa halbkreisförmigen Querschnittsprofil und mit einem Schaft (221;231) und einem Fußteil (222;232) das rückwärtige Schalenteil (220) ein zu dessen Schaft (221) winkelverstellbares Fußteil (222) und in dem dem Fußteil (222) gegenüberliegenden Wandbereich eine fensterartige Durchbrechung (280) mit einem in diese eingesetzten, austauschbaren plattenförmigen Winkeladapter (285) aufweist, der mit einer Eingriffvertiefung oder Eingrifföffnung (286) versehen ist, in die zur Winkelfestlegung zwischen dem Fußteil (222) und dem Schaft (221) des rückwärtigen Schalenteiles (220) ein Nocken (287) eingreift, der zur Fixierung einer vorbestimmten Winkelstellung in dem rückwärtigen Bereich des Fußteiles (222) an diesem angeformt ist.

23. Vorrichtung nach Anspruch 22, dadurch gekennzeichnet, daß in die fensterartige Durchbrechung (280) im rückwärtigen Schalenteil (220) Winkeladapter (285) mit unterschiedlichen Lagenanordnungen der Eingrifföffnungen (286,286',286",286"') für den Nocken (287) zur Winkeladapterfläche zur Fixierbarkeit des Fußteiles (222) zum Schaft (221) einsetzbar sind.

24. Vorrichtung nach einem der Ansprüche 22 und 23, dadurch gekennzeichnet, daß die fensterartige Durchbrechung (280) in der Wand des rückwärtigen Schalenteiles (220) etwa rechteckförmig ist und der Winkeladpater (285) eine der Form und den Abmessungen der fensterartigen Durchbrechung (280) entsprechende Ausgestaltung aufweist.

25. Vorrichtung nach Anspruch 23, dadurch gekennzeichnet, daß die Eingriffsöffnung (286") für den Nocken (287) im unteren Bereich des Winkeladapters (285) ausgebildet ist.

26. Vorrichtung nach Anspruch 23, dadurch gekennzeichnet, daß die Eingriffsöffnung (286') für den Nocken (287) im oberen Bereich des Winkeladapters (285) ausgebildet ist.

27. Vorrichtung nach einem der Ansprüche 22 bis 26, dadurch gekennzeichnet, daß der Winkeladapter (285) eine sich über die Adapterlänge erstreckende schlitzförmige Eingriffsöffnung (286"') für den Nocken (287) aufweist.

28. Vorrichtung nach einem der Ansprüche 22 bis 27, dadurch gekennzeichnet, daß der plattenförmige Winkeladapter (285) an seinem umlaufenden Rand (285a) mit einer umlaufenden oder sich über sich gegenüberliegende Bereiche erstreckende Nuten (288) zum Eingriff des die fensterartige Durchbrechung (280) begrenzenden Randes (280a) versehen ist.

29. Vorrichtung nach einem der Ansprüche 22 bis 28, dadurch gekennzeichnet, daß der plattenförmige Winkeladapter (285) aus einem federnd-elastischen Material, zweckmäßigerweise einem Kunststoff, besteht und mittels Klemmsitz am umlaufenden Rand (280a) der fensterartigen Durchbrechung (280) gehalten ist.

30. Vorrichtung nach einem der Ansprüche 22 bis 29, dadurch gekennzeichnet, daß der plattenförmige Winkeladapter (285) an seinen beiden Längsseitenkanten (285b,285c) keilförmig sich nach oben erweiternde Verstärkungen (289,289a) aus einem federnd-elastischen Material aufweist.

31. Vorrichtung nach einem der Ansprüche 22 bis 30, dadurch gekennzeichnet, daß der plattenförmige Winkeladapter (285) in seinem oberen Bereich zwei im Abstand voneinander angeordnete Durchbrechungen (283) zum Eingriff eines zangenartigen Öffnungswerkzeuges aufweist.

32. Vorrichtung nach einem der Ansprüche 22 bis 31, dadurch gekennzeichnet, daß der Nocken (287) als Feststellschraube ausgebildet ist.

33. Vorrichtung nach einem der vorangegangenen Ansprüche 1 bis 32, dadurch gekennzeichnet, daß von den beiden eine etwa L-Form aufweisenden Schalenteilen (320, 330) mit einem etwa halbkreisförmigen Querschnittsprofil und mit einem Schaft (321,331) und einem Fußteil (322,332) das vordere Schalenteil (330) ein zu dessen Schaft (331) winkelverstellbares Fußteil (332) und dem Schaft (331) eine Winkeleinstelleinrichtung (390) aufweist, vermittels der das Fußteil (332) zum Schaft (331) in einer vorgegebenen Winkelstellung arretierbar ist, wobei das rückwärtige Schalenteil (320) ein zu dessen Schaft (321) winkelverstellbares Fußteil (322) oder ein zu dessen Schaft (321) in einem vorgegebenen Winkel festgestelltes Fußteil (322) aufweist.

34. Vorrichtung nach Anspruch 33, dadurch gekennzeichnet, daß das zum Schaft (331) verstellbare Fußteil (332) des vorderen Schalenteiles (330) über einen quer zur Schaftlängsrichtung verlaufenden ziehharmonikabalgartig ausgebildeten Abschnitt (391) mit einer Anzahl von gefalteten, filmscharnierartig miteinander verbundenen Materialstreifen (391a) verbunden ist, wobei die das Fußteil (332) und den Schaft (331) miteinander verbindenden seitlich an dem Schalenkörper (10) ausgebildeten Schwenkgelenke (335) mit Winkelarretierungseinrichtungen versehen sind.

35. Vorrichtung nach Anspruch 33, dadurch gekennzeichnet, daß die Winkeleinstelleinrichtung (390) aus einem zwischen dem Fußteil (332) und dem Schaft (331) des vorderen Schalenteils (330) angeordneten, dem Außenprofil des Schalenkörpers (310) bzw. des vorderen Schalenteils (330) entsprechend geformten plattenförmigen Winkeladapter (395) besteht, der mittels Rast- und Verriegelungseinrichtungen (397) an dem Schaft (331) und dem Fußteil (332) des vorderen Schalenteils (330) lösbar gehalten ist.

36. Vorrichtung nach einem der Ansprüche 33 bis 35, dadurch gekennzeichnet, daß der plattenförmige Winkeladapter (395) eine Breite aufweist, aufgrund der das Fußteil (332) zu dem Schaft (331) des vorderen Schalenteils (330) eine Winkelstellung von 90° einnimmt.

37. Vorrichtung nach einem der Ansprüche 33 bis 36, dadurch gekennzeichnet, daß der plattenförmige Winkeladapter (395) eine Breite aufweist, aufgrund der das Fußteil (332) zu dem Schaft (331) des vorderen Schalenteils (330) eine Winkelstellung von 120° einnimmt.

38. Vorrichtung nach einem der Ansprüche 33 bis 37, dadurch gekennzeichnet, daß das Fußteil (332) und der Schaft (331) des vorderen Schalenteils (330) im Verbindungsbereich (339) eine fensterartige Durchbrechung (392) aufweist, die sich bis in den Seitenbereich des Fußteiles (332) und des Schaftes (331) erstreckt und in der der plattenförmige Winkeladapter (395) angeordnet ist.

39. Vorrichtung nach einem der Ansprüche 33 bis 38, dadurch gekennzeichnet, daß die gegenüberliegenden, quer zur Schaftlängsrichtung verlaufenden Ränder (392a,392b) der fensterartigen Durchbrechung (392) außenseitig wulstartige oder andersartig profilierte leistenförmige Verstärkungen (393) aufweisen und daß der plattenförmige Winkeladapter (395) an seinem oberen (395a) und unteren Rand (395b) mit den Verstärkungen (393) entsprechend ausgebildeten Gegenprofilen als Randprofile (396) versehen und mittels Klemmsitz an den Verstärkungen (393) an dem Fußteil (332) oder dem Schaft (331) des vorderen Schalenteils (330) gehalten ist.

40. Vorrichtung nach Anspruch 39, dadurch gekennzeichnet, daß die Randverstärkungen (393) an dem Fußteil (332) und dem Schaft (331) des vorderen Schalenteils (330) ein Querschnittsprofil mit einer quadratischen oder rechteckförmigen, konisch nach oben verlaufende Seitenflächen (393a,393b) aufweisenden Fläche (393c) aufweisen, und daß die Randprofile (396) an dem plattenförmigen Winkeladapter (395) als entsprechendes, die Randverstärkungen (393) an dem Fußteil (332) und dem Schaft (331) übergreifendes Gegenprofil (396') ausgebildet sind.

41. Vorrichtung nach einem der Ansprüche 33 bis 40, dadurch gekennzeichnet, daß zur Befestigung des plattenförmigen Winkeladapters (395) an dem Schaft (331) und dem Fußteil (332) des vorderen Schalenteils (330) der Winkeladapter (395) einendseitig (395c) und innenwandseitig mindestens zwei feststehende Verriegelungshaken (398), die in Durchbrechungen (398') in den Wandflächen des Schaftes (331) und des Fußteils (332) eingreifen, und an seinem anderen Ende (395d) mindestens zwei drehbare, als Exzenter ausgebildete Verriegelungshaken (399) aufweist, die in entsprechende Durchbrechungen (399') in den Wandflächen des Schaftes (331) und des Fußteiles (332) des vorderen Schalenteils (330) eingreifen.

42. Vorrichtung nach einem der Ansprüche 33 bis 41, dadurch gekennzeichnet, daß der plattenförmige Winkeladapter (395) aus einem federnd-elastischen Material, zweckmäßigerweise aus einem Kunststoff, besteht.

43. Vorrichtung nach einem der Ansprüche 1 bis 42, dadurch gekennzeichnet, daß von den beiden, eine etwa L-Form aufweisenden Schalenteilen (420,430) mit einem etwa halbkreisförmigen Querschnittsprofil das rückwärtige Schalenteil (420) unter Ausbildung mindestens eines abbiegbaren Abschnittes (445) vom oberen Schalenteilrand (420a) ausgehend schlitzförmige Durchbrechungen (440,540) aufweist, die sich über einen Bereich in Schalenteillängsrichtung derart erstrecken, daß in dem rückwärtigen Schalenteil (420) federnd-elastische und gegeneinander verschiebbare Wandabschnitte (425,426,445) ausgebildet sind, und daß eine diese Abschnitte (425,426, 445) fixierende Einrichtung (460) vorgesehen ist.

44. Vorrichtung nach Anspruch 43, dadurch gekennzeichnet, daß in dem rückwärtigen Schalenteil (420) zwei im Abstand, in Schalenteillängsrichtung verlaufende und sich vom oberen Schalenrand (420a) erstreckende schlitzförmige Durchbrechungen (440,540) ausgebildet sind, die in etwa U-förmige Abschnitte (441, 541) mit parallel zueinander verlaufenden schlitzförmigen Schenkelabschnitten (442,542) auslaufen, so daß in der Wand des rückwärtigen Schalenteiles (420) zwei zungenförmige Wandabschnitte (46,146) ausgebildet sind, wobei die zu den schlitzförmigen Durchbrechungen (40,140) benachbarten Wandabschnitte (25,26) feststehend sind.

45. Vorrichtung nach einem der Ansprüche 43 und 44, dadurch gekennzeichnet, daß der zwischen den schlitzförmigen Schenkelabschnitten (442,542) ausgebildetde Steg (445') federnd-elastisch an der Wand des rückwärtigen Schalenteils (420) gehalten ist.

46. Vorrichtung nach einem der Ansprüche 43 und 44, dadurch gekennzeichnet, daß der zwischen den schlitzförmigen Schenkelabschnitten (442,542) ausgebildete Steg (445') über eine Gelenkverbindung mit der Wand des rückwärtigen Schalenteiles (420) verbunden ist.

47. Vorrichtung nach einem der Ansprüche 43 bis 46, dadurch gekennzeichnet, daß zur Fixierung der gegeneinander verschiebbaren Wandabschnitte (425,426, 445,446,546) in Anpassung an die jeweilige Wadenform und -größe des Unterschenkels das rückwärtige Schalenteil (420) benachbart zu seinem oberen Rand (420a) eine Anzahl von schlitzförmigen Durchbrechungen (450) zur Aufnahme eines Verschlußorgans, bevorzugterweise eines Spanngurtes oder -bandes (460), vorgesehen ist.

48. Vorrichtung nach einem der Ansprüche 43 bis 46, dadurch gekennzeichnet, daß zur Fixierung der Wandabschnitte (425,426) des rückwärtigen Schalenteils (420) in diesen benachbart zum oberen Schalenteilrand (420a) schlitzförmige Durchbrechungen (450) zur Aufnahme von Verschlußorganen, bevorzugterweise von Spanngurten oder -bändern (460) vorgesehen sind.

49. Vorrichtung nach Anspruch 48, dadurch gekennzeichnet, daß die Verschlußorgane an den Wandabschnitten (425,426) des rückwärtigen Schalenteils (420) befestigt sind.

## Claims

1. Device for surrounding and securing extremities and regions thereof in the region of the lower leg and of the thigh comprising a shell body (10) made of two L-shaped shell parts (20, 30) which can be tensed the one against the other with an approximately semicircular cross section profile which encompass the extremity to be surrounded, whereby at least one exhaustable sleeve cushion is provided between the shell parts (20, 30) and the extremity, cushion in the inner part situated between both cushion walls of which a multitude of filling bodies movable the one with respect to the other is provided, characterized in that both shell parts (20, 30) have a grid structure extending over the whole wall surface of each shell part which is made of grid braces and/or grid ribs in longitudinal (11) and transverse ribs (12), whereby at least one longitudinal rib (11a) is provided in each shell part which extends substantially over the whole length of the shell part and which is configured at least as wide or with a bigger cross section than the remaining longitudinal braces, transverse braces or ribs (11, 12) and the junctions (13) of the transverse ribs (12) on the longitudinal rib (11a) which extends over the whole length are wider than the consecutive transverse rib part with a preferably diminishing width or diameter, that at least the rear shaft upper part (21) of the shell body (10) has a longitudinal slot (14) for the width or diameter adjustment, whereby the longitudinal ribs limiting the longitudinal slot (14) are adjustable and fixable to the desired distance over a detachable junction element (15 ; 115) and that the front shell part (30) has a foot part (32) swivellable to its shaft (31) over a joint (35) and the rear shell part (20) has a foot part (22) swivellable to its shaft (21) over a second joint (25), whereby the joint (35) and/or the second joint (25) can be stopped in any angular position by means of an angle adjusting device (90).

2. A device according to claim 1, characterized in that the grid braces and/or grid ribs (11, 12) have a width or a diameter between 1,5 cm and 3 cm.

3. A device according to any of the claims 1 and 2, characterized in that the grid braces and/or grid ribs (11, 12) have a respectively different width depending on the loads acting thereon and/or have a different diameter.

4. A device according to any of the claims 1 to 3, characterized in that the foot part (32) of the front shell part (30) which is adjustable with respect to the shaft (31) is connected over a section (91) configured in the manner of an accordion bellow running transversely to the shaft longitudinal direction with a great number of folded material stripes (91a) connected with each other in the manner of a film hinge, whereby the swivelling joints (35) which connect the foot part (32) and the shaft (31) the one with the other, which are configured laterally on the shell body (10), are provided with angle fixing devices.

5. A device according to any of the claims 1 to 3, characterized in that the angle adjustment device (90) is made of a rib-shaped, grid-shaped or plate-shaped angle adapter (95) placed between the foot part (32) and the shaft (31) of the front shell part (30), formed correspondingly to the outer profile of the shell body (10) or of the front shell part (30), adapter which is detachably held on the shaft (31) and the foot part (32) of the front shell part (30) by means of snap-in and locking devices (97).

6. A device according to claim 5, characterized in that the rib-shaped, grid-shaped or plate-shaped angle adapter (95) has a width by reason of which the foot part (32) takes up an angular position of 90° to the shaft (31) of the front shell part (30).

7. A device according to claim 5, characterized in that the rib-shaped, grid-shaped or plate-shaped angle adapter (95) has a width by reason of which the foot part (32) takes up an angular position of 120° to the shaft (31) of the front shell part (30).

8. A device according to any of the claims 1 to 7, characterized in that the foot part (32) and the shaft (31) of the front shell part (30) has in the junction area (39) a window-type opening (92) which extends up to the side area of the foot part (32) and of the shaft (31) and in which the rib-shaped, grid-shaped or plate-shaped angle adapter (95) is placed.

9. A device according to claim 8, characterized in that the opposing edges (92a, 92b) running transversely to the shaft longitudinal direction (92) of the window-type opening (92) have on the outside bead-type reinforcements (93) or ledge-type reinforcements which have another profile and that the rib-shaped, grid-shaped or plate-shaped angle adapter (95) is provided on its upper (95a) and on its lower edge (95b) with counterprofiles configured correspondingly to the reinforcements (93) as edge profiles (96) and is held press-fitted on the reinforcements (93) on the foot part (32) or the shaft (31) of the front shell part (30).

10. A device according to claim 9, characterized in that the edge reinforcements (93) on the foot part (32) and the shaft (31) of the front shell part (30) have a cross section profile with a square or rectangular surface (93c) which has upwards tapered side faces (93a, 93b) and that the edge profiles (96) are configured on the rib-shaped, grid-shaped or plate-shaped angle adapter (95) as a corresponding counterprofile (96') overlapping the edge reinforcements (93) on the foot part (32) and the shaft (31).

11. A device according to any of the claims 5 to 10, characterized in that, for fixing the rib-shaped, grid-shaped or plate-shaped angle adapter (95) on the shaft (31) and the foot part (32) of the front shell part (30), the angle adapter (95) has at the one end (95c) and on the inner face at least two fixed locking hooks (98) which engage into openings (98') in the wall faces of the shaft (31) and of the foot part (32) and at its other end (95d) at least two rotating locking hooks (99) configured as eccentrics which engage into corresponding openings (99') in the wall faces of the shaft (31) and of the foot part (32) of the front shell part (30).

12. A device according to any of the claims 5 to 11, characterized in that the rib-shaped, grid-shaped or plate-shaped angle adapter (95) is made of a resilient elastic material, appropriately of plastics.

13. A device according to any of the claims 1 to 12, characterized in that both shell parts (20, 30) and/or the angle adjusting device (90) are made of plastics.

14. A device according to any of the claims 1 to 13, characterized in that a sleeve cushion or pad is placed on the shell part inner sides, cushion in which a multitude of filling bodies movable the one to the other is contained.

15. A device according to any of the claims 1 to 14, characterized in that guides (16', 17') framing the longitudinal slot (14) are adjustable and fixable to the desired distance over a detachable junction element (15 ; 115), whereby the junction element is a slide (15 ; 115) with edge profiles partially overlapping the circumference of the guides or engaging into the guide.

16. A device according to claim 15, characterized in that one of the two guides (16', 17') rises above the other in direction of the shell upper part longitudinal axis by a length (a) which is at least as big as the length (1) of the slide or of the junction element, whereby one of the two guides (16') is configured substantially up to the shell upper part edge (18).

17. A device according to claim 15, characterized in that the detachable junction element or the slide (15 ; 115) is displaceable by means of a stop limited in direction of the shaft upper part edge (18 ; 118).

18. A device according to any of the claims 1 to 17, characterized in that the shaft upper part (10) has two substantially parallel running ribs or bead-shaped elevations (16, 17) which engage into correspondingly formed grooves of the slide which is displaceable in the longitudinal direction but which is secured against a lateral removal.

19. A device according to claim 18, characterized in that the guides (16', 17') is a dovetail guiding.

20. A device according to any of the claims 1 to 19, characterized in that the junction element or the slide (15 ; 115) grasps over two longitudinal ribs of the shaft upper part and can be guided along this part.

21. A device according to any of the claims 1 to 20, characterized in that the spaced longitudinal ribs which form an intermediate space are provided with elevations (16, 17) which form bead-type guiding rails (16', 17').

22. A device according to any of the claims 1 to 21, characterized in that, among the two shell parts (220 ; 230) which have an approximately L-shape with an approximately semicircular cross section profile and with a shaft (221 ; 231) and a foot part (222 ; 232), the rear shell part (220) has a foot part (222), the angle of which is adjustable with respect to its shaft (221) and in the wall area opposed to the foot part (222) a window-type opening (280) with a replaceable plate-shaped angle adapter (285) placed therein which is provided with an engagement recess or an engagement opening (286) into which a cam (287) engages for determining the angle between the foot part (222) and the shaft (221) of the rear shell part (220), this cam being moulded in the rear area of the foot part (222) thereon for fixing a predetermined angular position.

23. A device according to claim 22, characterized in that angle adapters (285) with different position placements of the engagement openings (286, 286', 286", 286"') for the cam (287) with respect to the angle adapter surface can be placed into the window-type opening (280) in the rear shell part (220) for allowing the fixing of the foot part (222) to the shaft (221).

24. A device according to any of the claims 22 and 23,
characterized in that the window-type opening (280) in the wall of the rear shell part (220) is approximately rectangular and the angle adapter (285) has a configuration corresponding to the shape and to the dimensions of the window-type opening (280).

25. A device according to claim 23, characterized in that the engagement opening (286") for the cam (287) is configured in the lower area of the angle adapter (285).

26. A device according to claim 23, characterized in that the engagement opening (286') for the cam (287) is configured in the upper area of the angle adapter (285).

27. A device according to any of the claims 22 to 26, characterized in that the angle adapter (285) has a slot-shaped engagement opening (286"') for the cam (287) which extends over the adapter length.

28. A device according to any of the claims 22 to 27, characterized in that the plate-shaped angle adpater (285) is provided, on its peripheral edge (285a), with a peripheral groove or with grooves (288) extending over opposing areas for the engagement of the edge (280a) limiting the window-type opening (280).

29. A device according to any of the claims 22 to 28, characterized in that the plate-shaped angle adapter (285) is made of a resilient elastic material, appropriately of plastics, and is held press fitted on the peripheral edge (280a) of the window-type opening (280).

30. A device according to any of the claims 22 to 29, characterized in that the plate-shaped angle adapter (285) has wedge-shaped upwards enlarging reinforcements (289, 289a) made of a resilient elastic material on its both longitudinal side edges (285b, 285c).

31. A device according to any of the claims 22 to 30, characterized in that the plate-shaped angle adapter (285) has two openings (283) placed spaced from each other in its upper area for the engagement of a pliers-type opening tool.

32. A device according to any of the claims 22 to 31, characterized in that the cam (287) is configured as a locking screw.

33. A device according to any of the preceding claims 1 to 32, characterized in that, among the two shell parts (320 ; 330) which have an approximately L-shape with an approximately semicircular cross section profile and with a shaft (321 ; 331) and a foot part (322, 332), the front shell part (330) has a foot part (332), the angle of which is adjustable to its shaft (331) and the shaft (331) an angle adjusting device (390) by means of which the foot part (332) can be locked to the shaft (331) in a predetermined angular position, whereby the rear shell part (320) has a foot part (322) the angle of which is adjustable to its shaft (321) or a foot part (322) fixed to its shaft (321) in a predetermined angle.

34. A device according to claim 33, characterized in that the foot part (332) of the front shell part (330) adjustable to the shaft (331) is connected over a section (391) configured in the manner of an accordion bellow running transversely to the shaft longitudinal direction with a great number of folded material stripes (391a) connected with each other in the manner of a film hinge, whereby the swivelling joints (335) which connect the foot part (332) and the shaft (331) the one with the other, which are configured laterally on the shell body (10), are provided with angle fixing devices.

35. A device according to claim 33, characterized in that the angle adjusting device (390) is made of a plate-shaped angle adapter (395) placed between the foot part (332) and the shaft (331) of the front shell part (330) formed correspondingly to the outer profile of the shell body (310) or of the front shell part (330), adapter which is detachably held on the shaft (331) and the foot part (332) of the front shell part (330) by means of snap-in and locking devices (397).

36. A device according to any of the claims 33 to 35, characterized in that the plate-shaped angle adapter (395) has a width by reason of which the foot part (332) takes up an angular position of 90° to the shaft (331) of the front shell part (330).

37. A device according to any of the claims 33 to 36, characterized in that the plate-shaped angle adapter (395) has a width by reason of which the foot part (332) takes up an angular position of 120° to the shaft (331) of the front shell part (330).

38. A device according to any of the claims 33 to 37, characterized in that the foot part (332) and the shaft (331) of the front shell part (330) has a window-type opening (392) in the junction area (339) which extends into the side area of the foot part (332) and of the shaft (331) and in which the plate-shaped angle adapter (395) is placed.

39. A device according to any of the claims 33 to 38, characterized in that the opposing edges (392a, 392b) of the window-type opening (392) running transversely to the shaft longitudinal direction have on the outside bead-type reinforcements (393) or ledge-type reinforcements which have another profile and that the plate-shaped angle adapter (395) is provided on its upper (395a) and on its lower edge (395b) with counterprofiles configured correspondingly to the reinforcements (393) as edge profiles (e96) and is held press-fitted on the reinforcements (393) on the foot part (332) or the shaft (331) of the front shell part (330).

40. A device according to claim 39, characterized in that the edge reinforcements (393) on the foot part (332) and the shaft (331) of the front shell part (330) have a cross section profile with a square or rectangular surface (393c) which has upwards tapered side faces (393a, 393b) and that the edge profiles (396) are configured on the plate-shaped angle adapter (395) as a corresponding counterprofile (396') overlapping the edge reinforcements (393) on the foot part (332) and the shaft (331).

41. A device according to any of the claims 33 to 40, characterized in that, for fixing the plate-shaped angle adapter (395) on the shaft (331) and the foot part (332) of the front shell part (330), the angle adapter (395) has at the one end (395c) and on the inner wall face at least two fixed locking hooks (398) which engage into openings (398') in the wall faces of the shaft (331) and of the foot part (332) and at its other end (395d) at least two rotating locking hooks (399) configured as eccentrics which engage into corresponding openings (399') in the wall faces of the shaft (331) and of the foot part (332) of the front shell part (330).

42. A device according to any of the claims 33 to 41, characterized in that the plate-shaped angle adapter (395) is made of a resilient elastic material, appropriately of plastics.

43. A device according to any of the claims 1 to 42, characterized in that, among the two shell parts (420, 430) which have an approximately L-shape with an approximately semicircular cross section profile, the rear shell part (420) has slit-shaped openings (440, 540) starting from the upper shell part edge (420a) by constituting at least one section (445) which can be bent off, these openings extending over an area in the shell part longitudinal direction, that resilient elastic wall sections (425, 426, 445) which are displaceable against each other are configured in the rear shell part (420) and that a device (460) fixing these sections (425, 426, 445) is provided for.

44. A device according to claim 43, characterized in that two spaced slit-shaped openings (440, 450) running in the shell part longitudinal direction which extend from the upper shell edge (420a) are configured in the reart shell part (420), these openings ending in approximately U-shaped sections (441, 541) with slit-shaped leg sections (442, 542) running parallel to each other so that two tongue-shaped wall sections (46, 146) are configured in the wall of the rear shell part (420), whereby the wall sections (25, 26) adjacent to the slit-shaped openings (40, 140) are fixed.

45. A device according to any of the claims 43 and 44,
characterized in that the web (445') configured between the slit-shaped leg sections (442, 542) is held resiliently elastically on the wall of the rear shell part (420).

46. A device according to any of the claims 43 and 44,
characterized in that the web (445') configured between the slit-shaped leg sections (442, 542) is connected over a link joint with the wall of the rear shell part (420).

47. A device according to any of the claims 43 to 46, characterized in that, for fixing the wall sections (425, 426, 445, 446, 546) which are displaceable the one against the other in adaptation to the respective calf shape and to the size of the lower leg, the rear shell part (420) has, adjacent to its upper edge (420a), a number of slit-shaped openings (450) for receiving a closing element, preferably a tightening belt or tightening strap (460).

48. A device according to any of the claims 43 to 46, characterized in that, for fixing the wall sections (425, 426) of the rear shell part (420), slit-shaped openings 8450) are provided in these wall sections adjacent to the upper shell part edge (420a) for receiving closing elements, preferably tightening belts or tightening straps (460).

49. A device according to claim 48, characterized in that the closing elements are fixed on the wall sections (425, 426) of the rear shell part (420).

## Revendications

1. Dispositif permettant d'entourer et de fixer des extrémités et des zones d'extrémités dans la zone de la jambe et de la cuisse constitué par un corps de coque (10) constitué par deux parties de coque (20, 30) en forme de L, qui peuvent être serrées l'une contre l'autre, avec un profil de section à peu près en forme de demi-cercle, qui entourent l'extrémité à entourer, au moins un coussin à manchette dans lequel le vide peut être fait étant prévu entre les parties de coque (20, 30) et l'extrémité, coussin dans l'intérieur situé entre les deux parois de coussin duquel une multitude de corps de remplissage mobiles les uns par rapport aux autres est prévue, caractérisé en ce que les deux parties de coque (20, 30) présentent une structure de grille, qui s'étend sur toute la surface des parois de chacune des parties de coque, qui est constituée par des montants de grille et/ou des nervures de grille dans des nervures longitudinales (11) et transversales (12), au moins une nervure longitudinale (11a) étant prévue par partie de coque, nervure qui s'étend substantiellement sur toute la longueur de la partie de coque et qui est configurée au moins aussi large que ou avec une section plus grande que les autres montants longitudinaux, montants transversaux ou nervures transversales (11, 12) et les endroits de raccordement (13) des nervures transversales (12) sur la nervure longitudinale (11a) qui s'étend sur toute la longueur sont plus larges que la partie de nervure transversale qui se rattache, qui est de préférence configurée en diminuant dans la largeur ou le diamètre, qu'au moins la partie supérieure arrière de la tige (21) du corps de coque (10) présente une fente longitudinale (14) pour l'ajustage de la largeur ou du diamètre, les nervures longitudinales qui délimitent la fente longitudinale (14) étant réglables et fixables à l'écart souhaité par un élément de jonction amovible (15 ; 115) et que la partie de coque antérieure (30) présente une partie pied (32) pivotante par rapport à sa tige (31) par l'intermédiaire d'une articulation (35) et la partie de coque postérieure (20) présente une partie pied (22) pivotante par rapport à sa tige (21) par l'intermédiaire d'une seconde articulation (25), l'articulation (35) et/ou la seconde articulation (25) étant bloquable (s) dans n'importe quelle position angulaire au moyen d'un dispositif de réglage d'angle (90).

2. Dispositif selon la revendication 1, caractérisé en ce que les montants de grille et/ou nervures de grille (11, 12) ont une largeur ou un diamètre entre 1,5 cm et 3 cm.

3. Dispositif selon l'une des revendications 1 et 2, caractérisé en ce que les montants de grille et/ou nervures de grille (11, 12) sont de largeur respectivement différente en fonction des sollicitations agissant sur eux/elles et/ou ont un diamètre différent.

4. Dispositif selon l'une des revendications 1 à 3, caractérisé en ce que la partie pied (32) de la partie de coque antérieure (30), partie pied réglable par rapport à la tige (31), est reliée par une section (91) configurée de type soufflet d'accordéon qui est transversale par rapport au sens longitudinal de la tige à un certain nombre de bandes de matière (91a) pliées reliées les unes aux autres à la manière d'une charnière en film, les articulations pivotantes (35), qui relient la partie pied (32) et la tige (31) l'une à l'autre, qui sont configurées latéralement sur le corps de coque (10) étant pourvues de dispositifs de blocage d'angle.

5. Dispositif selon l'une des revendications 1 à 3, caractérisé en ce que le dispositif de réglage d'angle (90) est constitué par un adaptateur d'angle (95) en forme de nervure, de grille ou de plaque placé entre la partie pied (32) et la tige (31) de la partie de coque antérieure (30), formé de manière à correspondre au profil extérieur du corps de coque (10) ou de la partie de coque antérieure (30), adaptateur qui est maintenu amovible sur la tige (31) et la partie pied (32) de la partie de coque antérieure (30) au moyen de dispositifs d'enclenchement et de verrouillage (97).

6. Dispositif selon la revendication 5, caractérisé en ce que l'adaptateur d'angle en forme de nervure, de grille ou de plaque (95) a une largeur en raison de laquelle la partie pied (32) prend par rapport à la tige (31) de la partie de coque antérieure (30) une position angulaire de 90°.

7. Dispositif selon la revendication 5, caractérisé en ce que l'adaptateur d'angle en forme de nervure, de grille ou de plaque (95) a une largeur en raison de laquelle la partie pied (32) prend par rapport à la tige (31) de la partie de coque antérieure (30) une position angulaire de 120°.

8. Dispositif selon l'une des revendications 1 à 7, caractérisé en ce que la partie pied (32) et la tige (31) de la partie de coque antérieure (30) présente, dans la zone de jonction (39), une découpure de type fenêtre (92) qui s'étend jusque dans la zone latérale de la partie pied (32) et de la tige (31) et dans laquelle l'adaptateur d'angle en forme de nervure, de grille ou de plaque (95) est placé.

9. Dispositif selon la revendication 8, caractérisé en ce que les bords (92a, 92b) de la découpure de type fenêtre (92), opposés et transversaux par rapport au sens longitudinal de la tige, présentent sur le côté extérieur des renforcements (93) de type bourrelet ou profilés d'un autre type en forme de baguette et que l'adaptateur d'angle en forme de nervure, de grille ou de plaque (95) est pourvu, sur son bord supérieur (95a) et inférieur (95b), de profilés antagonistes configurés de manière à correspondre aux renforcements (93) comme profils de bord (96) et est maintenu par ajustement pressé sur les renforcements (93) sur la partie pied (32) ou sur la tige (31) de la partie de coque antérieure (30).

10. Dispositif selon la revendication 9, caractérisé en ce que les renforcements de bord (93) sur la partie pied (32) et la tige (31) de la partie de coque antérieure (30) présentent un profil de section avec une surface carrée ou rectangulaire (93c) qui présente des faces latérales devenant coniques vers le haut (93a, 93b) et que les profilés de bord (96) sur l'adaptateur d'angle en forme de nervure, de grille ou de plaque (95) sont configurés comme profilé antagoniste (96') correspondant qui enveloppe les renforcements de bord (93) sur la partie pied (32) et la tige (31).

11. Dispositif selon l'une des revendications 5 à 10, caractérisé en ce que, pour la fixation de l'adaptateur d'angle en forme de nervure, de grille ou de plaque (95) à la tige (31) et à la partie pied (32) de la partie de coque antérieure (30), l'adaptateur d'angle (95) présente, à l'une de ses extrémités (95c) et sur la face de la paroi intérieure, au moins deux crochets de verrouillage fixes (98) qui s'engrènent dans des découpures (98') dans les faces de paroi de la tige (31) et de la partie pied (32) et, à son autre extrémité (95d), au moins deux crochets de verrouillage rotatifs (99), configurés comme des excentriques, qui s'engrènent dans des découpures correspondantes (99') dans les faces de paroi de la tige (31) et de la partie pied (32) de la partie de coque antérieure (30).

12. Dispositif selon l'une des revendications 5 à 11, caractérisé en ce que l'adaptateur d'angle en forme de nervure, de grille ou de plaque (95) est en une matière élastique à la manière d'un ressort, de manière appropriée en plastique.

13. Dispositif selon l'une des revendications 1 à 12, caractérisé en ce que les deux parties de coque (20, 30) et/ou le dispositif d'ajustage d'angle (90) sont en plastique.

14. Dispositif selon l'une des revendications 1 à 13, caractérisé en ce qu'un coussin à manchette ou un rembourrage, dans lequel une multitude de corps de remplissage mobiles les uns par rapport aux autres est contenue, est placé sur les faces intérieures des parties de coque.

15. Dispositif selon l'une des revendications 1 à 14, caractérisé en ce que des guides (16', 17') encadrant la fente longitudinale (14) sont réglables et fixables à l'écart souhaité par un élément de jonction amovible (15 ; 115), l'élément de jonction étant un coulisseau (15 ; 115) avec des profilés de bord qui enveloppent partiellement les guides sur la périphérie ou qui s'engrènent dans le guidage.

16. Dispositif selon la revendication 15, caractérisé en ce que l'un des deux guides (16', 17') dépasse l'autre dans le sens de l'axe longitudinal de la partie supérieure de coque d'une longueur (a) qui est au moins aussi grande que la longueur (1) du coulisseau ou de l'élément de jonction, l'un des deux guides (16') étant configuré substantiellement jusqu'au bord de la partie supérieure de coque (18).

17. Dispositif selon la revendication 15, caractérisé en ce que l'élément de jonction amovible ou le coulisseau (15 ; 115) est déplaçable de manière limitée en direction du bord de la partie supérieure de tige (18 ; 118) au moyen d'une butée.

18. Dispositif selon l'une des revendications 1 à 17, caractérisé en ce que la partie supérieure de la tige (10) présente deux nervures allant substantiellement parallèlement ou deux rehaussements en forme de bourrelet (16, 17) qui s'engrènent dans des rainures formées de manière correspondante du coulisseau qui est déplaçable dans le sens de la longueur mais qui est bloqué contre un enlèvement latéral.

19. Dispositif selon la revendication 18, caractérisé en ce que les guides (16', 17') sont un guidage en queue d'aronde.

20. Dispositif selon l'une des revendications 1 à 19, caractérisé en ce que l'élément de jonction ou le coulisseau (15 ; 115) a prise sur deux nervures longitudinales de la partie supérieure de tige et peut être guidé le long de celle-ci.

21. Dispositif selon l'une des revendications 1 à 20, caractérisé en ce que les nervures longitudinales, formant un espace intermédiaire, sont pourvues de rehaussements (16, 17) de type bourrelet, formant des rails de guidage (16', 17').

22. Dispositif selon l'une des revendications 1 à 21, caractérisé en ce que, parmi les deux parties de coque présentant une forme approximativement en forme de L (220 ; 230) avec un profil de section approximativement en forme de demi-cercle et avec une tige (221 ; 331) et une partie pied (222 ; 232), la partie de coque postérieure (220) présente une partie pied (222) à angle réglable par rapport à sa tige (221) et une découpure de type fenêtre (280) dans la zone de paroi opposée à la partie pied (222) avec un adaptateur d'angle en forme de plaque (285) échangeable mis en place dans cette découpure, adaptateur qui est pourvu d'un enfoncement d'engrènement ou d'une ouverture d'engrènement (286) dans laquelle une came (287) s'engrène pour fixer l'angle entre la partie pied (222) et la tige (221) de la partie de coque postérieure (220), came qui est moulée dans la zone postérieure de la partie pied (222) sur celle-ci pour fixer une position angulaire prédéterminée.

23. Dispositif selon la revendication 22, caractérisé en ce que des adaptateurs d'angle (285) avec différents positionnements des ouvertures d'engrènement (286, 286', 286", 286"') pour la came (287) par rapport à la surface de l'adaptateur d'angle pour la fixabilité de la partie pied (222) par rapport à la tige (221) peuvent être mis en place dans la découpure de type fenêtre (280) dans la partie de coque postérieure (220).

24. Dispositif selon l'une des revendications 22 et 23, caractérisé en ce que la découpure de type fenêtre (280) dans la paroi de la partie de coque postérieure (220) est approximativement rectangulaire et l'adaptateur d'angle (285) présente une configuration qui correspond à la forme et aux dimensions de la ouverture de type fenêtre (280).

25. Dispositif selon la revendication 23, caractérisé en ce que l'ouverture d'engrènement (286") pour la came (287) est configurée dans la zone inférieure de l'adaptateur d'angle (285).

26. Dispositif selon la revendication 23, caractérisé en ce que l'ouverture d'engrènement (286') pour la came (287) est configurée dans la zone supérieure de l'adaptateur d'angle (285).

27. Dispositif selon l'une des revendications 22 à 26, caractérisé en ce que l'adaptateur d'angle (285) présente une ouverture d'engrènement (286"') en forme de fente pour la came (287), ouverture qui s'étend sur la longueur de l'adaptateur.

28. Dispositif selon l'une des revendications 22 à 27, caractérisé en ce que l'adaptateur d'angle en forme de plaque (285) est pourvu, sur son bord périphérique (285a), d'une rainure périphérique ou de rainures (288) qui s'étendent sur des zones opposées pour l'engrènement du bord (280a) qui délimite la découpure de type fenêtre (280).

29. Dispositif selon l'une des revendications 22 à 28, caractérisé en ce que l'adaptateur d'angle en forme de plaque (285) est en une matière élastique à la manière d'un ressort, de manière appropriée en plastique, et est maintenu par ajustement pressé sur le bord périphérique (280a) de la découpure de type fenêtre (280).

30. Dispositif selon l'une des revendications 22 à 29, caractérisé en ce que l'adaptateur d'angle en forme de plaque (285) présente, sur ses deux arêtes de côté longitudinal (285b, 285c), des renforcements qui s'élargissent en forme de coin vers le haut (289, 289a) en une matière élastique à la manière d'un ressort.

31. Dispositif selon l'une des revendications 22 à 30, caractérisé en ce que l'adaptateur d'angle en forme de plaque (285) présente, dans sa zone supérieure, deux découpures (283) placées à un certain écart l'une de l'autre pour l'engrènement d'un outil d'ouverture de type tenaille.

32. Dispositif selon l'une des revendications 22 à 31, caractérisé en ce que la came (287) est configurée comme une vis de blocage.

33. Dispositif selon l'une des revendications précédentes 1 à 32, caractérisé en ce que, parmi les deux parties de coque présentant une forme approximativement en forme de L (320, 330) avec un profil de section approximativement en forme de demi-cercle et avec une tige (321, 331) et une partie pied (322, 332), la partie de coque antérieure (330) présente une partie pied (332) à angle réglable par rapport à sa tige (331) et la tige (331) un dispositif de réglage d'angle (390) au moyen duquel la partie pied (332) peut être bloquée par rapport à la tige (331) dans une position angulaire prédéterminée, la partie de coque postérieure (320) présentant une partie pied (322) à angle ajustable par rapport à sa tige (321) ou une partie pied (322) bloquée par rapport à sa tige (321) dans un angle prédéterminé.

34. Dispositif selon la revendication 33, caractérisé en ce que la partie pied (332) réglable par rapport à la tige (331) de la partie de coque antérieure (330) est reliée à un certain nombre de bandes de matière (391a) pliées reliées l'une à l'autre à la manière d'une charnière film par une section (391) configurée de type soufflet d'accordéon transversale par rapport au sens longitudinal de la tige, les articulations pivotantes (335) reliant la partie pied (332) et la tige (331) l'une à l'autre, configurées latéralement sur le corps de coque (10), étant pourvues de dispositifs de blocage d'angle.

35. Dispositif selon la revendication 33, caractérisé en ce que le dispositif de réglage d'angle (390) est constitué par un adaptateur d'angle (395) en forme de plaque placé entre la partie pied (332) et la tige (331) de la partie de coque antérieure (330), formée de manière correspondante au profil extérieur du corps de coque (310) ou de la partie de coque antérieure (330), adaptateur qui est maintenu amovible sur la tige (331) et la partie pied (332) de la partie de coque antérieure (330) au moyen de dispositifs d'enclenchement et de verrouillage (397).

36. Dispositif selon l'une des revendications 33 à 35, caractérisé en ce que l'adaptateur en forme de plaque (395) présente une largeur en raison de laquelle la partie pied (332) prend une position angulaire de 90° par rapport à la tige (331) de la partie de coque antérieure (330).

37. Dispositif selon l'une des revendications 33 à 36, caractérisé en ce que l'adaptateur en forme de plaque (395) présente une largeur en raison de laquelle la partie pied (332) prend une position angulaire de 120° par rapport à la tige (331) de la partie de coque antérieure (330).

38. Dispositif selon l'une des revendications 33 à 37, caractérisé en ce que la partie pied (332) et la tige (331) de la partie de coque antérieure (330) présente, dans la zone de jonction (339), une découpure de type fenêtre (392) qui s'étend jusque dans la zone latérale de la partie pied (332) et de la tige (331) et dans laquelle l'adaptateur d'angle en forme de plaque (395) est placé.

39. Dispositif selon l'une des revendications 33 à 38, caractérisé en ce que les bords opposés (392a, 392b) de la découpure de type fenêtre (392) transversaux par rapport au sens longitudinal de la tige présentent, sur le côté extérieur, des renforcements (393) de type bourrelet ou profilés d'un autre type en forme de baguette et que l'adaptateur d'angle en forme de plaque (395) est pourvu, sur son bord supérieur (395a) et inférieur (395b), de profilés antagonistes configurés de manière à correspondre aux renforcements (393) comme profils de bord (396) et est maintenu par ajustement pressé sur les renforcements (393) sur la partie pied (332) ou sur la tige (331) de la partie de coque antérieure (330).

40. Dispositif selon la revendication 39, caractérisé en ce que les renforcements de bord (393) sur la partie pied (332) et la tige (331) de la partie de coque antérieure (330) présentent un profil de section avec une surface carrée ou rectangulaire qui présente des faces latérales devenant coniques vers le haut (393a, 393b) et que les profilés de bord (396) sur l'adaptateur d'angle en forme de plaque (395) sont configurés comme profilé antagoniste (396') correspondant qui enveloppe les renforcements de bord (393) sur la partie pied (332) et la tige (331).

41. Dispositif selon l'une des revendications 33 à 40, caractérisé en ce que, pour la fixation de l'adaptateur d'angle en forme de plaque (395) à la tige (331) et à la partie pied (332) de la partie de coque antérieure (330), l'adaptateur d'angle (395) présente, à l'une de ses extrémités (395c) et sur la face de la paroi intérieure, au moins deux crochets de verrouillage fixes (398) qui s'engrènent dans des découpures (398') dans les faces de paroi de la tige (331) et de la partie pied (332) et, à son autre extrémité (395d), au moins deux crochets de verrouillage rotatifs (399), configurés comme des excentriques, qui s'engrènent dans des découpures correspondantes (399') dans les faces de paroi de la tige (331) et de la partie pied (332) de la partie de coque antérieure (330).

42. Dispositif selon l'une des revendications 33 à 41, caractérisé en ce que l'adaptateur d'angle en forme de plaque (395) est en une matière élastique à la manière d'un ressort, de manière appropriée en plastique.

43. Dispositif selon l'une des revendications 1 à 42, caractérisé en ce, parmi les deux parties de coque présentant une forme approximativement en forme de L (420, 430) avec un profil de section approximativement en forme de demi-cercle, la partie de coque postérieure (420) présente, en formant au moins une section qui peut être recourbée (445), des découpures en forme de fente (440, 540) qui partent du bord supérieur de la partie de coque (420a) qui s'étendent sur une zone dans le sens de la longueur de la partie de coque de telle manière que des sections de paroi (425, 426, 445) élastiques à la manière d'un ressort et déplaçables l'une contre l'autre sont formées dans la partie de coque postérieure (420) et qu'un dispositif (460) qui fixe ces sections (425, 426, 445) est prévu.

44. Dispositif selon la revendication 43, caractérisé en ce que deux découpures en forme de fentes (440, 450), qui vont dans le sens de la longueur de la partie de coque à un certain écart l'une de l'autre et qui s'étendent à partir du bord supérieur de la coque (420a), sont configurées dans la partie de coque postérieure (420), découpures qui se terminent en sections approximativement en forme d'U (441, 541) avec des sections de montants en forme de fentes parallèles l'une à l'autre (442, 542) si bien que deux sections de paroi en forme de languette (46, 146) sont configurées dans la paroi de la partie de coque postérieure (420), les sections de paroi (25, 26) voisines des découpures en forme de fentes (40, 140) étant fixes.

45. Dispositif selon l'une des revendications 43 et 44, caractérisé en ce que la barrette (445') configurée entre les sections de montant en forme de fentes (442, 542) est maintenue élastiquement à la manière d'un ressort sur la paroi de la partie de coque postérieure (420).

46. Dispositif selon l'une des revendications 43 et 44, caractérisé en ce que la barrette (445') configurée entre les sections de montant en forme de fentes (442, 542) est reliée par une jonction articulée à la paroi de la partie de coque postérieure (420).

47. Dispositif selon l'une des revendications 43 à 46, caractérisé en ce que, pour la fixation des sections de paroi déplaçables l'une contre l'autre (425, 426, 445, 446, 546) en adaptation à la forme du mollet respectif et à la taille de la jambe, la partie de coque postérieure (420) présente, au voisinage de son bord supérieur (420a), un certain nombre de découpures en forme de fentes (450) pour recevoir un organe de fermeture, de préférence une sangle de serrage ou une bande de serrage (460).

48. Dispositif selon l'une des revendications 43 à 46, caractérisé en ce que, pour la fixation des sections de paroi (425, 426) de la partie de coque postérieure (420), des découpures en forme de fentes (450) sont prévues dans celles-ci au voisinage du bord supérieur de la partie de coque (420a) pour recevoir des organes de fermeture, de préférence des sangles de serrage ou des bandes de serrage (460).

49. Dispositif selon la revendication 48, caractérisé en ce que les organes de fermeture sont fixés aux sections de paroi (425, 426) de la partie de coque postérieure (420).
